# EUROPEAN PATENT APPLICATION

(11) **EP 4 781 943 A2**
(43) Date of publication of application: **29.07.2026**
(21) Application number: 26184685.1
(22) Date of filing: 14.03.2023
(51) Int. Cl.: A61B 90/00

(54) **SYSTEMS FOR THE ENDOVASCULAR TREATMENT OF HYDROCEPHALUS AND ELEVATED INTRACRANIAL PRESSURE**

(30) Priority: 16.03.2022 US 202263320621 P
(62) Divisional of application: 23715394.5
(71) Applicant: CereVasc, Inc., Charlestown, MA 02129 (US)
(72) Inventor: Calderan, Joseph, Charlestown, MA 02129 (US); Hawkes, Emma, Charlestown, MA 02129 (US); Hern, Kim, Charlestown, MA 02129 (US); Maiorano, Anthony, Charlestown, MA 02129 (US); Rezac, David, Charlestown, MA 02129 (US); Sattell, Jack, Charlestown, MA 02129 (US); Wirth, Alexandra, Charlestown, MA 02129 (US)
(74) Representative: Gillespie, Richard

(57) **Abstract**

The present disclosure is directed towards a shroud assembly for delivering an implant through a catheter lumen, the shroud assembly comprising: an elongate tubular shroud body having open proximal and distal ends; an implant retention feature coupled to the proximal end of the shroud body, the implant retention feature having a proximal end and an open distal end; and a shroud lumen extending through the shroud body from the distal end opening of the shroud body, the proximal end opening of the shroud body, and an interior of the implant retention feature, respectively, and wherein the implant retention feature is configured to secure a proximal end portion of the implant in the shroud assembly when the implant is disposed in the shroud lumen and the shroud assembly is disposed in the catheter lumen.

## Description

### RELATED APPLICATION

This application claims the benefit of U.S. Provisional Patent Application Serial No. 63/320,621, filed on March 16, 2022, the entire disclosure of which is expressly incorporated by reference herein.

### FIELD OF THE INVENTION

The inventions disclosed herein relate to improved systems and methods for accessing subarachnoid spaces, and for draining cerebrospinal fluid (CSF), (e.g., to relieve elevated intracranial pressure or treat normal pressure hydrocephalus), using an endovascular approach. More particularly, the present disclosure pertains to systems and methods for treatment of hydrocephalus, pseudotumor cerebri, and/or intracranial hypertension.

### BACKGROUND

There has been significant progress made in recent decades in the development and practice of minimally invasive surgical devices and procedures. As used herein, "minimally invasive" refers to the use of surgical devices and implants that access the body via the vasculature via arterial or venous access in the groin, arm or neck area, as opposed to more invasive traditional procedures that access the body more directly through percutaneous/solid tissue incisions and cutting and/or boring through bones, as needed to access the internal area of the body on which the procedure is performed. Minimally invasive may also refer to the to the use of surgical devices and implants that access the body via other natural body orifices, cavities and tubular structures, such as the esophagus, intestines, bronchial passageways, etc. Percutaneous access through the skin into solid tissue, e.g., for accessing the liver, prostate or lungs, e.g., using a trocar and stylet, may also be considered minimally invasive.

Examples of minimally invasive surgical devices and procedures to relieve elevated intracranial pressure or treat normal pressure hydrocephalus are described in United States Patents Nos. 10,272,230 and 10,765,846 and United States Patent Application Publication No. US20200030588A1, the entire disclosures of which are incorporated herein by reference, as though set forth in full.

There is an ongoing need, however, to improve minimally invasive neurosurgical devices that can access the intracranial subarachnoid space ("SAS") and deploy devices designed to drain cerebrospinal fluid from the SAS to the venous system. The complexity of the device preparation steps, impact of the preparation steps to the devices, and time required to prepare the minimally invasive system for clinical use are important considerations, and it is desirable to simplify and complete such preparation in less than an hour, and preferably less than 15 minutes. Improvements to enhance the user experience by simplifying the device deployment procedure and mitigating risks to patient safety during the minimally invasive procedure are also needed. Further, improvements are needed to enhance the manufacturability of the minimally invasive neurosurgical devices such that the devices can be manufactured at reduced costs and at larger volumes to enable commercialization of the technology.

### SUMMARY

A delivery system for deployment of an implant is provided, the delivery system including a delivery catheter having a first delivery catheter lumen extending from an open distal end of the delivery catheter into a handle coupled to a proximal end portion of the delivery catheter, a tissue penetrating element disposed on, and extending distally from, an open distal end thereof. The delivery system further includes a guard member having a proximal portion disposed over, and translatable relative to, the respective tissue penetrating element and distal end of the delivery catheter, the guard member defining a guard member lumen configured to receive and cover the tissue penetrating element, the guard member lumen defining a longitudinal axis of the guard member. The proximal portion of the guard member transitions into a split-open distal portion adjacent a distal opening of the guard member lumen, the split-open distal portion comprising a contour configured to engage and deflect the tissue penetrating element at an angle relative to the longitudinal axis of the guard member when the guard member is translated proximally relative to the tissue penetrating element, and
wherein the split-open distal portion of the guard member comprises arcuate sloping portions on opposing sides of the contour, the sloping portions having respective surfaces that are configured to engage and deflect the tissue penetrating element. The contour of the split-open distal portion of the guard member is preferably configured to allow the respective sloping portions of the opposing sides to open and collapse around the tissue penetrating element when the respective guard member and delivery catheter are retracted into a guide catheter.

The delivery system may further include a pull wire coupled to the guard member and configured to translate the guard member proximally relative to the tissue penetrating element. The pull wire may have a rectangular, circular, or crescent cross-sectional profile.Also, the pull wire bifurcates into respective first and second pull wire members. The pull wire further includes an arcuate radiopaque marker embedded within the guard member adjacent the distal opening of the guard member lumen, wherein the first and second pull wire members are each attached to the arcuate radiopaque marker. Additionally, the guard member includes a second guard member lumen that spans between the proximal and distal portions of the guard member, wherein the second guard member lumen is configured to receive an elongate guide member therethrough over which the guard member translates, and wherein the second guard member lumen is partially surrounded by the arcuate marker.

Additionally, the delivery catheter includes a second delivery catheter lumen, wherein the pull wire is disposed in the second delivery catheter lumen, and wherein the handle further comprises a flush port in fluid communication with the first catheter lumen, and a tether actuation mechanism, wherein the proximal end of the pull wire is coupled to the tether actuation mechanism, and wherein the tether actuation mechanism is configured to be pulled relative to the handle to thereby translate the guard member relative to the tissue penetrating element to thereby expose the tissue penetrating element.

Optionally, a distal portion of the pull wire is embedded within the guard member. An arcuate radiopaque marker may be embedded within the guard member adjacent the distal opening of the guard member lumen. The guard member, having a first guard member lumen, is also provided with a second guard member lumen that spans between the proximal and distal portions of the guard member, wherein the second guard member lumen is configured to receive an elongate guide member therethrough over which the guard member translates, and wherein the second guard member lumen is partially surrounded by the arcuate marker.

Optionally, an elongate radiopaque marker spine is embedded in the tissue penetrating element, wherein the marker spine is aligned substantially parallel to a longitudinal axis of the tissue penetrating element, and wherein, when the tissue penetrating element is disposed within the guard member lumen, the arcuate radiopaque marker embedded in the guard member and the radiopaque marker spine embedded in the tissue penetrating element are configured to indicate a position, orientation and trajectory of the tissue penetrating element.

Also, a delivery system for deployment of an implant is provided, the delivery system including a delivery catheter having a proximal opening, a distal opening, and delivery catheter lumen extending therebetween. The delivery system further includes a shroud assembly, the shroud assembly including an elongate tubular shroud body having open proximal and distal ends, an implant retention feature coupled to the proximal end of the shroud body, the implant retention feature having a proximal end and an open distal end, and a shroud lumen extending through the shroud body from the distal end opening of the shroud body, the proximal end opening of the shroud body, and an interior of the implant retention feature, respectively. The delivery catheter lumen is configured to receive the shroud assembly therein and allow passage of the shroud assembly therethrough, and the implant retention feature is configured to secure a proximal end portion of an implant in the shroud assembly when the implant is disposed in the shroud lumen and the shroud assembly is disposed in the delivery catheter lumen. The implant retention feature is configured to release the proximal end portion of the implant when the shroud assembly is advanced out of the delivery catheter lumen through the distal opening of the delivery catheter. A delivery wire may be coupled to the proximal end of the implant retention feature and configured to advance or retract the shroud assembly within the delivery catheter lumen with the implant disposed within the shroud lumen.

Optionally, the implant retention feature comprises a plurality of elongated members extending between two annular members, wherein the elongated members comprise respective protrusions facing radially outward, such that, when the implant is disposed within the shroud lumen, and the shroud assembly is disposed within the delivery catheter lumen, the protrusions are compressed by an interior wall of the delivery catheter lumen and the elongated members are in-turn compressed against the proximal end portion of the implant to thereby grasp the implant within the shroud lumen.

Additionally, the shroud body comprises a spiral cut hypotube and an uncut axial spine disposed along a length of the hypotube. Also, the shroud assembly includes a proximal radiopaque marker disposed at the proximal end of the implant retention feature, and a distal radiopaque marker disposed at the distal end opening of the shroud body. The distal radiopaque marker includes an annular edge configured to contact an annular edge of an implant distal anchoring mechanism in order to advance the implant through the delivery catheter lumen and out the distal end opening of the delivery catheter.

Additionally, a transfer device is provided for flushing and transferring an implant into a delivery catheter, the implant transfer device including an elongate housing, an implant transfer lumen extending through the housing from a proximal opening in the housing to a distal opening in the housing, a distal luer connector coupled to the distal opening, a touhy-borst adapter and stopcock coupled to the proximal opening, and an annular passive seal is disposed within the implant transfer lumen, wherein the annular passive seal has a lumen configured to retain an expandable portion of the implant in a compressed configuration, and wherein the distal luer connector, implant transfer lumen, passive seal lumen, touhy-borst adapter and stopcock are in fluid communication.

Additionally, the transfer device may have a shroud assembly disposed in the implant transfer lumen. The shroud assembly may have an elongate tubular shroud body having open proximal and distal ends, an implant retention feature coupled to the proximal end of the shroud body, the implant retention feature having a proximal end and an open distal end, and a shroud lumen extending through the shroud body from the distal end opening of the shroud body, the proximal end opening of the shroud body, and an interior of the implant retention feature, respectively, wherein a body portion of the implant is disposed within the shroud lumen, a proximal portion of the implant is disposed within the retention feature, and a distal anchoring mechanism of the implant is disposed in a compressed configuration within the passive seal lumen, and wherein the transfer device distal luer connector, implant transfer lumen, passive seal lumen, a lumen of the implant, and transfer device touhy-borst adapter and stopcock are in fluid communication.

Optionally, the shroud assembly further has a delivery wire coupled to the proximal end of the implant retention feature and configured to advance or retract the shroud assembly within the implant transfer lumen with the implant disposed within the shroud lumen. Also, the implant retention feature may have a plurality of elongated members extending between two annular members, wherein the elongated members include respective protrusions facing radially outward. The shroud body may have a spiral cut hypotube and an uncut axial spine disposed along a length of the hypotube. The shroud assembly further having a proximal radiopaque marker disposed at the proximal end of the implant retention feature, and a distal radiopaque marker disposed at the distal end opening of the shroud body. Optionally, the transfer device further have a tubular support member disposed in the implant transfer lumen, the tubular support member defining an inner lumen through which the shroud assembly and implant carried in the shroud assembly are advanced, wherein a distal portion of the tubular support member tapers radially inward in a distal direction, such that an inner diameter of the tubular support member lumen decreases in a distal direction along a length of the distal portion, and such that, as the shroud assembly and implant are advanced distally through tubular support member lumen, the shroud retention feature is compressed to thereby secure or grasp a proximal end portion of the implant.

An assembly for verifying patency of a shunt implant prior to clinical use is provided, the assembly including a base, a three-way stopcock secured to the base, the stopcock having a plurality of luer fittings, a first stopcock luer fitting connectable to a fluid source (e.g., a syringe), a fluid column in fluid communication with a second stopcock luer fitting, and a flushing line having a distal end opening in fluid communication with a third stopcock luer fitting, the flushing line further having a proximal end opening.

The stopcock has a first open position that places the fluid source in fluid communication with the flushing line, a second open position that places the fluid source in fluid communication with the fluid column, and a third open position that places the fluid column in fluid communication with the flushing line. The base may be provided with a hinge mechanism secured to the stopcock, wherein the hinge mechanism is configured to swivel the stopcock and fluid column from a first position in which the fluid column is disposed substantially parallel to a surface of the base, to a second position in which the fluid column is disposed substantially perpendicular to the base surface. The hinge mechanism may be configured to releasably secure the stopcock in the second position.

A method for preparing a shunt implant for clinical use is provided, wherein the method includes connecting the flushing line of the above-described assembly to the distal luer connector of the above-described implant transfer device, so that the flushing line is in fluid communication with the shunt lumen. The stopcock is then placed in the first open position, and fluid from the fluid source is delivered through the respective flushing line and implant lumen. The stopcock is then placed in the second open position, and fluid from the fluid source is delivered into the fluid column. The stopcock is then placed in the third open position, and fluid flow from the fluid column through the respective flushing line and shunt lumen may be confirmed by observing fluid flowing out of the implant transfer device stopcock. The passive annular seal of the implant transfer device directs fluid flow in the implant transfer device lumen into the shunt lumen. Additionally, the flushing line may be disconnected from the distal luer connector of the implant transfer device, the distal luer connector of the implant transfer device is then connected to the proximal luer connector of a delivery catheter handle, and the shunt implant may be advanced through the implant transfer device lumen and into a lumen of the delivery catheter. The fluid source may be a syringe and the fluid may be heparinized saline.

Optionally, advancing the shunt implant through the implant transfer device lumen and into a lumen of a delivery catheter includes advancing the implant and shroud assembly with the pusher wire. Also, when advancing the implant and shroud assembly from the transfer device lumen into the delivery catheter lumen, a plurality of protrusions of the proximal retention feature compress the retention feature elongate members to grasp the proximal portion of the implant. Additionally, when advancing the implant and shroud assembly through the transfer device lumen and into the delivery catheter lumen, an annular edge of the shroud distal radiopaque marker of claim 25 contacts an annular edge of an implant distal anchoring mechanism to advance the implant through the transfer tool lumen into the delivery catheter lumen.

A catheter is provided having a first catheter lumen extending from an open distal end of the catheter into a handle coupled to a proximal end portion of the catheter, a tissue penetrating element disposed on, and extending distally from, an open distal end of the catheter, and a guard member having a proximal portion disposed over, and translatable relative to, the respective tissue penetrating element and distal end of the catheter, the guard member defining a guard member lumen configured to receive and cover the tissue penetrating element, the guard member lumen defining a longitudinal axis of the guard member, wherein the proximal portion of the guard member transitions into a split-open distal portion adjacent a distal opening of the guard member lumen, the split-open distal portion comprising a contour configured to engage and deflect the tissue penetrating element at an angle relative to the longitudinal axis of the guard member when the guard member is translated proximally relative to the tissue penetrating element. The split-open distal portion of the guard member may have arcuate sloping portions on opposing sides of the contour, the sloping portions having respective surfaces that are configured to engage and deflect the tissue penetrating element. The contour of the split-open distal portion of the guard member is configured to allow the respective sloping portions of the opposing sides to open and collapse around the tissue penetrating element when the respective guard member and catheter are retracted into a guide catheter. Also, the catheter may have a pull wire coupled to the guard member and configured to translate the guard member proximally relative to the tissue penetrating element. The pull wire comprises a rectangular, circular, or crescent cross-sectional profile. The distal portion of the pull wire bifurcates into respective first and second pull wire members. Also, the catheter may have an arcuate radiopaque marker embedded within the guard member adjacent the distal opening of the guard member lumen, wherein the first and second pull wire members are each attached to the arcuate radiopaque marker.

Additionally, in this the catheter, the guard member lumen includes a first guard member lumen, the guard member further having a second guard member lumen that spans between the proximal and distal portions of the guard member, wherein the second guard member lumen is configured to receive an elongate guide member therethrough over which the guard member translates, and wherein the second guard member lumen is partially surrounded by the arcuate marker. Optionally, the catheter further includes a second catheter lumen, wherein the pull wire is disposed in the second delivery catheter lumen, and wherein the handle further has a flush port in fluid communication with the first catheter lumen, and a tether actuation mechanism, wherein the proximal end of the pull wire is coupled to the tether actuation mechanism, and wherein the tether actuation mechanism is configured to be pulled relative to the handle to thereby translate the guard member relative to the tissue penetrating element to thereby expose the tissue penetrating element. Also, the distal portion of the pull wire is embedded within the guard member. The pull wire further including an arcuate radiopaque marker embedded within the guard member adjacent the distal opening of the guard member lumen. The guard member lumen having a first guard member lumen, the guard member further having a second guard member lumen that spans between the proximal and distal portions of the guard member, wherein the second guard member lumen is configured to receive an elongate guide member therethrough over which the guard member translates, and wherein the second guard member lumen is partially surrounded by the arcuate marker. Additionally, the catheter may have an elongate radiopaque marker spine embedded in the tissue penetrating element, wherein the marker spine is aligned substantially parallel to a longitudinal axis of the tissue penetrating element, and wherein, when the tissue penetrating element is disposed within the guard member lumen, the arcuate radiopaque marker embedded in the guard member and the radiopaque marker spine embedded in the tissue penetrating element are configured to indicate a position, orientation and trajectory of the tissue penetrating element.

A shroud assembly for delivering an implant through a catheter lumen is provided. The shroud assembly having an elongate tubular shroud body having open proximal and distal ends, an implant retention feature coupled to the proximal end of the shroud body, the implant retention feature having a proximal end and an open distal end, and a shroud lumen extending through the shroud body from the distal end opening of the shroud body, the proximal end opening of the shroud body, and an interior of the implant retention feature, respectively, and wherein the implant retention feature is configured to secure a proximal end portion of the implant in the shroud assembly when the implant is disposed in the shroud lumen and the shroud assembly is disposed in the catheter lumen. The implant retention feature is configured to release the proximal end portion of the implant when the shroud assembly is advanced out of a distal end opening of the catheter lumen. Also, the shroud assembly may have a delivery wire coupled to the proximal end of the implant retention feature and configured to advance or retract the shroud assembly within the delivery catheter lumen with the implant disposed within the shroud lumen.

Optionally, the implant retention feature may have a plurality of elongated members extending between two annular members, wherein the elongated members comprise respective protrusions facing radially outward, such that, when the implant is disposed within the shroud lumen, and the shroud assembly is disposed within the catheter lumen, the protrusions are compressed by an interior wall of the catheter lumen and the elongated members are in-turn compressed against the proximal end portion of the implant to thereby grasp the implant within the shroud lumen. The shroud body may have a spiral cut hypotube and an uncut axial spine disposed along a length of the hypotube. The shroud assembly may also have a proximal radiopaque marker disposed at the proximal end of the implant retention feature, and a distal radiopaque marker disposed at the distal end opening of the shroud body.

Other and further aspects and features of embodiments will become apparent from the ensuing detailed description in view of the accompanying figures.

### BRIEF DESCRIPTION OF THE DRAWINGS

FIG. 1 is a side view of a delivery assembly according to embodiments of the disclosed inventions;
FIGS. 2A-D are perspective, top and side views of a delivery catheter having a penetrating element guard, according to embodiments of the disclosed inventions;
FIGS. 3A-F are side, perspective and cross-sectional views of guard member and pull wire, according to embodiments of the disclosed inventions;
FIGS. 4A-B are side views of the interface between a delivery catheter having a penetrating element and the guard member of FIGS. 3A-F, according to embodiments of the disclosed inventions;
FIGS. 5A-C are down-the-barrel views of the interface between a delivery catheter having the penetrating element and the guard member of FIGS. 3A-F and FIGS. 4A-B, according to embodiments of the disclosed inventions;
FIGS. 6A-B are side and perspective views the distal portion of the delivery catheter having the penetrating element and FIGS. 6C-N are top and side views of alternative embodiments of the penetrating element;
FIGS. 7A-D are side views a shunt/implant and a shroud, according to embodiments of the disclosed inventions;
FIGS. 8A-F are side and detailed views of the actuation of the shroud with the shunt/implant, according to embodiments of the disclosed inventions;
FIGS. 9A-E are perspective and cross-sectional views of the shroud, according to alternative embodiments of the disclosed inventions;
FIGS. 10A-E are perspective, detailed and cross-sectional views of a shunt/implant, according to embodiments of the disclosed inventions;
FIGS. 11A-C are perspective and down-the-barrel views of an anchor, a proximal radiopaque marker, and an elongate guide member, according to embodiments of the disclosed inventions;
FIGS. 12A-B are perspective and down-the-barrel views of the proximal radiopaque marker of the anchor of FIGS. 11A-C, according to embodiments of the disclosed inventions;
FIG. 13 is a cross-sectional view of a handle for the delivery catheter of FIG. 16A, according to embodiments of the disclosed inventions;
FIGS. 14A-D are perspective and cross-sectional views of an embodiment of the handle for the delivery catheter of FIG. 2, according to embodiments of the disclosed inventions;
FIGS. 15A-F are perspective, cross-sectional and detailed views of transfer devices, according to embodiments of the disclosed inventions; FIG. 15G is a cross-sectional vew of a passive seal, according to embodiments of the disclosed inventions;
FIG. 16A is a perspective view of the transfer device of FIGS. 15A-C coupled to the handle of FIG. 13, and FIG. 16B is a perspective view of the transfer device of FIGS. 15D-G coupled to the handle of FIGS. 14A-D; according to embodiments of the disclosed inventions;
FIGS. 17A-B are perspective and top views of a reservoir assembly and the transfer device of FIGS. 15A-C and 16A, according to embodiments of the disclosed inventions; and
FIG. 18 is a flow chart of a patency test, according to embodiments of the disclosed inventions.

### DETAILED DESCRIPTION

For the following defined terms, these definitions shall be applied, unless a different definition is given in the claims or elsewhere in this specification.

All numeric values are herein assumed to be modified by the term "about," whether or not explicitly indicated. The term "about" generally refers to a range of numbers that one of skilled in the art would consider equivalent to the recited value (i.e., having the same function or result). In many instances, the terms "about" may include numbers that are rounded to the nearest significant figure.

The recitation of numerical ranges by endpoints includes all numbers within that range (e.g., 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

Various embodiments are described hereinafter with reference to the figures. The figures are not necessarily drawn to scale, the relative scale of select elements may have been exaggerated for clarity, and elements of similar structures or functions are represented by like reference numerals throughout the figures. It should also be understood that the figures are only intended to facilitate the description of the embodiments, and are not intended as an exhaustive description of the invention or as a limitation on the scope of the invention, which is defined only by the appended claims and their equivalents. In addition, an illustrated embodiment needs not have all the aspects or advantages shown. An aspect or an advantage described in conjunction with a particular embodiment is not necessarily limited to that embodiment and can be practiced in any other embodiments even if not so illustrated.

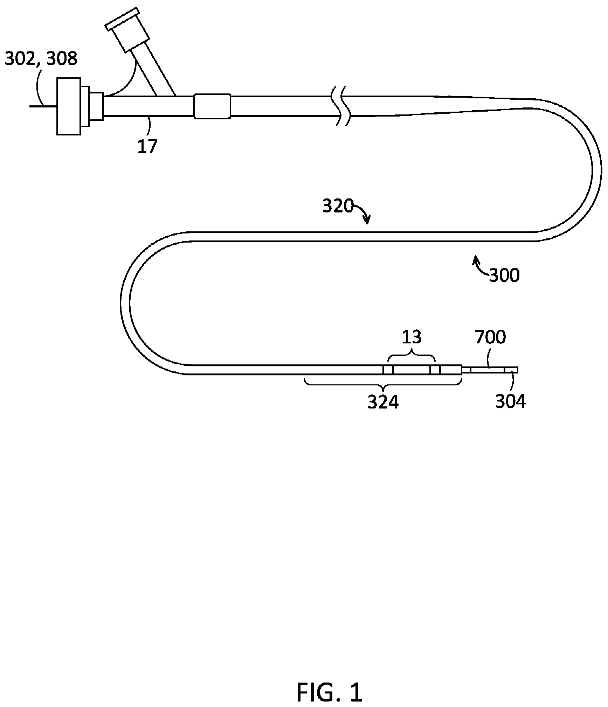

**FIG. 1** is a side view of a delivery assembly 300 for accessing the subarachnoid space and/or delivering an endovascular cerebrospinal fluid (CSF) implant (e.g., shunt, implant/shunt) into a target site of a patient, in accordance with embodiments of the disclosed inventions. The delivery assembly 300 may include an anchor 700 (not shown) and the shunt (not shown) detachably coupled to the delivery assembly 300. The delivery assembly 300 and the shunt may be composed of suitable biocompatible materials. The delivery assembly 300 is dimensioned to reach remote locations of the vasculature and is configured to deliver the shunt percutaneously to the target location (e.g., cerebellopontine angle cistern (CP angle cistern), inferior petrosal sinus (IPS)). The delivery assembly 300 includes a tubular member interface having an outer tubular member 320 (i.e., guide catheter) and an inner tubular member 304 (i.e., delivery catheter / micro catheter) coaxially disposed within the outer tubular member 320 and movable relative to the outer tubular member 320. The delivery assembly 300 may include a guidewire 302 coaxially disposed within the guide catheter 320 and/or the delivery catheter 304. The guidewire 302 can be, for example, 0.035" (0.889 mm) in diameter. Additionally to the guidewire 302, the delivery assembly 300 may include a delivery wire 308 disposed within the delivery catheter 304. The delivery wire 308 has a smaller diameter (e.g., approximately 0.010" (0.254 mm) to 0.018" (0.4572 mm) or other suitable dimension to facilitate accessing intracranial venous vasculature with other components of delivery assembly 300) compared to guidewire 302.

The guide catheter 320, delivery catheter 304, and guidewires 302/308 **(****FIG. 1****)** may be formed of suitable biocompatible materials, and may include markings 13 for purposes of imaging (e.g., markers composed of radio-opaque materials). Various known and often necessary accessories to the delivery assembly 300, e.g., one or more radiopaque marker bands 13 at the distal portion 324 of the guide catheter 320 to allow viewing of the position of the distal portion under fluoroscopy and a Luer assembly 17 for guidewires and/or fluids access, are shown in **FIG. 1****.** The delivery assembly 300 and/or the shunt may include a penetrating element (not shown) configured to pierce and/or penetrate a venous sinus wall (e.g., IPS wall and arachnoid layer to access a CP angle cistem). The delivery catheter 304, with or without a delivery guide wire, facilitates navigation and delivery of the shunt through the patient's vasculature to a target deployment location.

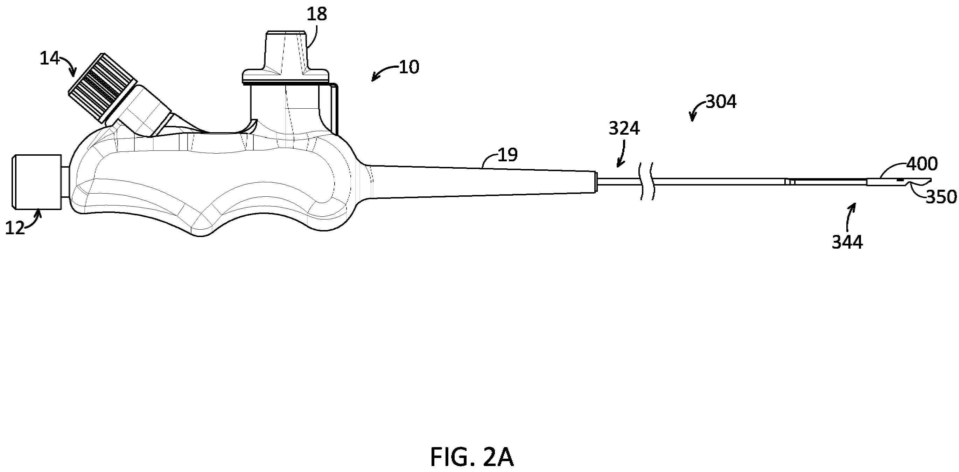

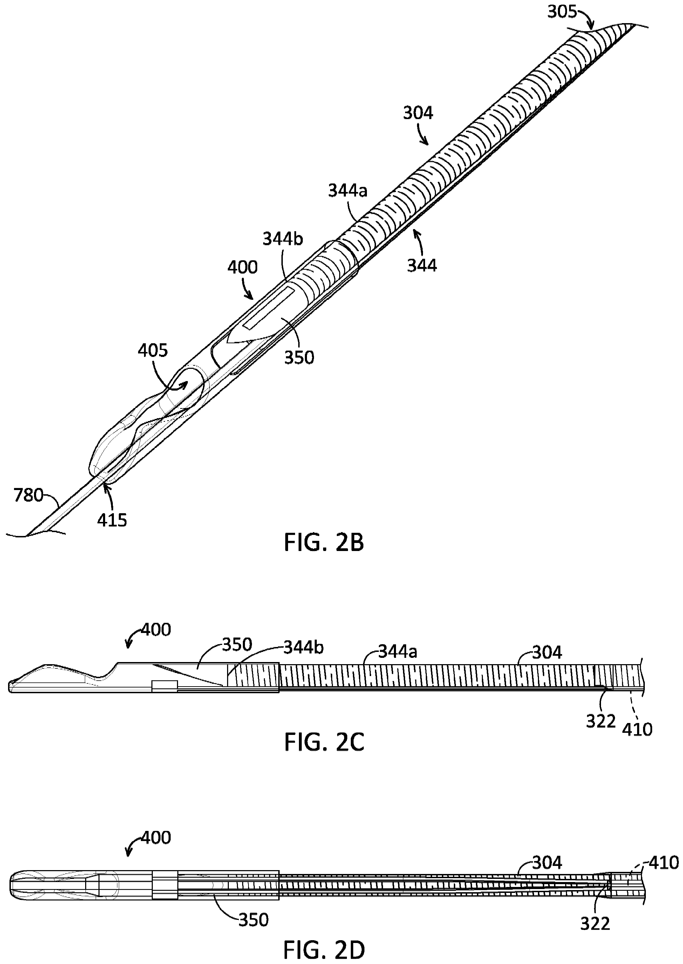

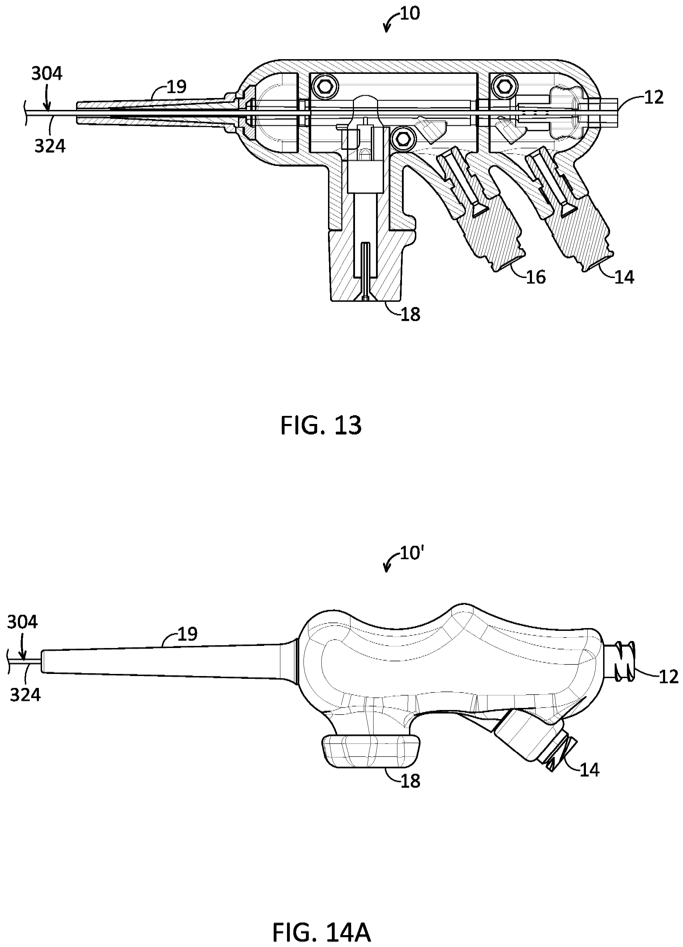

**FIG. 2A** illustrates a side view of the delivery catheter 304 according to another embodiment of the inventions. Instead of the Luer assembly 17 of **FIG. 1****,** the delivery catheter 304 is coupled and/or integrated to a handle 10, as shown in **FIG. 2A****.** The handle 10 comprises 10 a Luer fitting 12, a delivery lumen flush port 14, a tether retraction mechanism 18, and a strain relief 19; which will be further described in **FIGS. 13-14C** below. The delivery catheter 304 comprises a proximal portion 324; the proximal portion 324 may be coupled and/or integrated to the handle 10 through the strain relief 19. The delivery catheter 304 further comprises a distal portion 344 (detailed view of **FIG. 2B****);** the distal portion 344 comprises a penetrating element 350 disposed within a guard member 400, which will be further described in FIGS. 2B**-D.** Embodiments of delivery catheter 304 can include a mutli-layer construction. For example, the delivery catheter 304 can be manufactured by having a reinforcing member, such as a hypotube with regions of different flexibility (e.g., interrupted spiral cut, selective slots, fenestrations or their like), where the reinforcing member interior surface is coated, dipped or covered by a lubricious material, such as PTFE lining, and the reinforcing member exterior surface is coated, dipped or covered by a polymeric jacket, as described in U.S. Patent Nos. 10,272,230 and 10,765,846 and U.S. Patent App. Pub. No. US20200030588A1.

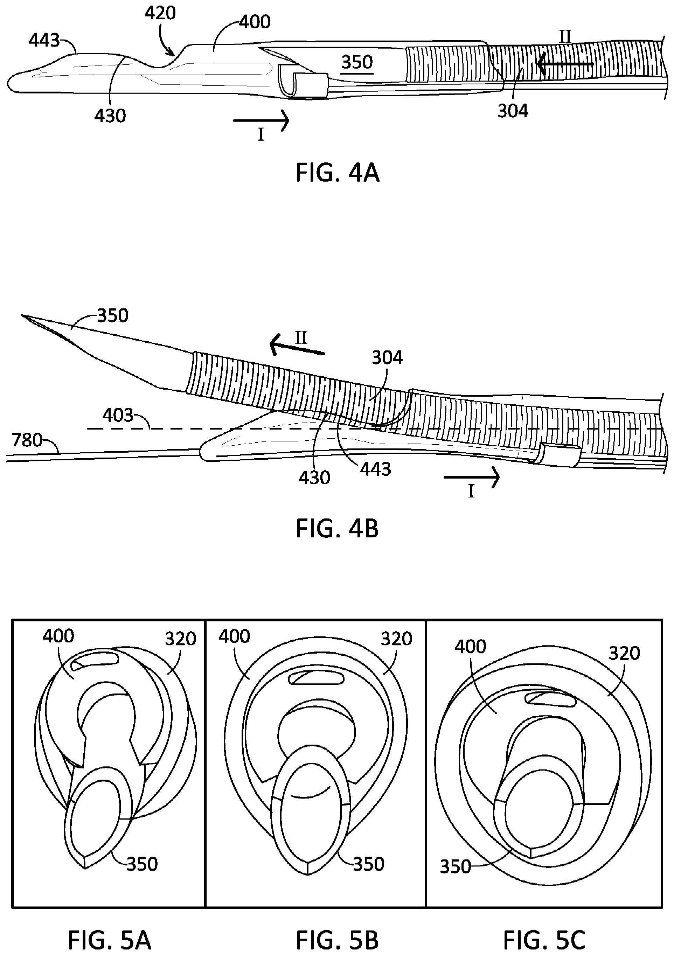

FIGS. 2B**-D** illustrate a perspective, side and top views of the distal portion 344 of the delivery catheter 304 of the delivery assembly 300, according to embodiments of the invention. The delivery assembly 300 comprises the delivery catheter 304 and the penetrating element guard or guard member 400. In some embodiments, the distal portion 344 of delivery catheter 304 comprises an elongated tubular shaft 344a and a distal end 344b, where the distal end 344b is coupled to or comprises the penetrating element 350. As shown in FIGS. 2A**-D,** the guard member 400 is configured to cover the penetrating element 350; the penetrating element 350 disposed within the guard member 400 is further illustrated in **FIG. 4A****.** The guard member 400 covers the penetrating element 350 during navigation of the delivery catheter 304 through the patient's vasculature to the target penetration site (e.g., a venous sinus wall). Further, the guard member 400 is configured to collapse around the shaft 344a of the delivery catheter 304 during withdrawal of the catheter 304, as described and depicted in FIGS. 5A**-C.** In alternative embodiments, the guard member 400 may cover the penetrating element 350 during withdrawal of delivery catheter 304 after accessing the subarachnoid space or shunt deployment, thereby preventing inadvertent puncture or damage to other components of delivery assembly (e.g., guide catheter) and/or the patient's vasculature. As shown in **FIG. 2B****,** the delivery catheter 304 comprises a first lumen 305 configured to receive and/or accommodate a shunt. The guard member 400 includes a first lumen 405 configured to receive the penetrating element 350 and a second lumen 415 configured to receive and/or accommodate an elongate guide member 780 as shown in FIG. 2B.

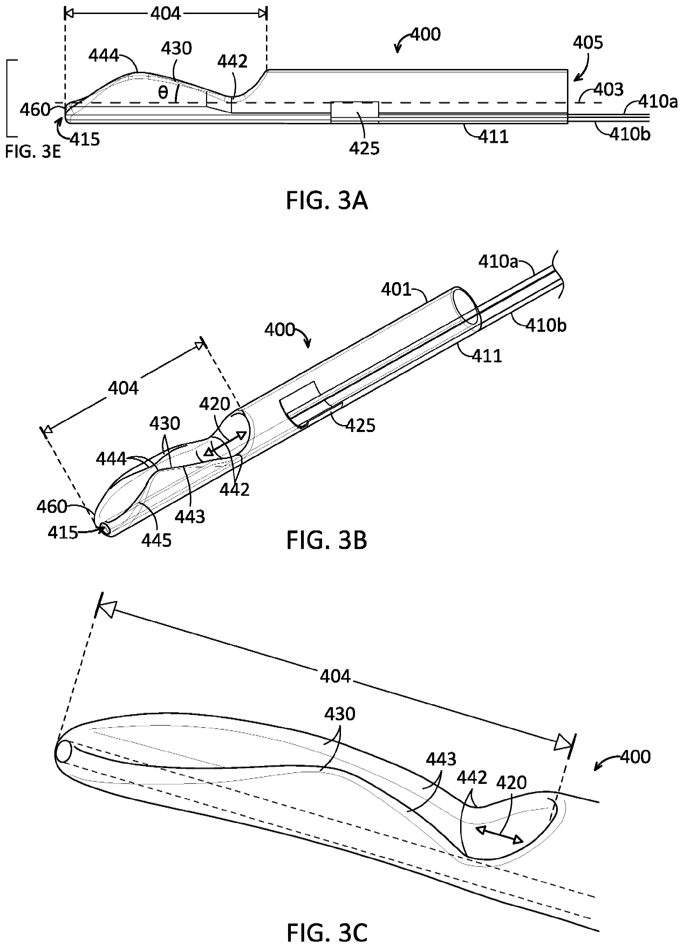

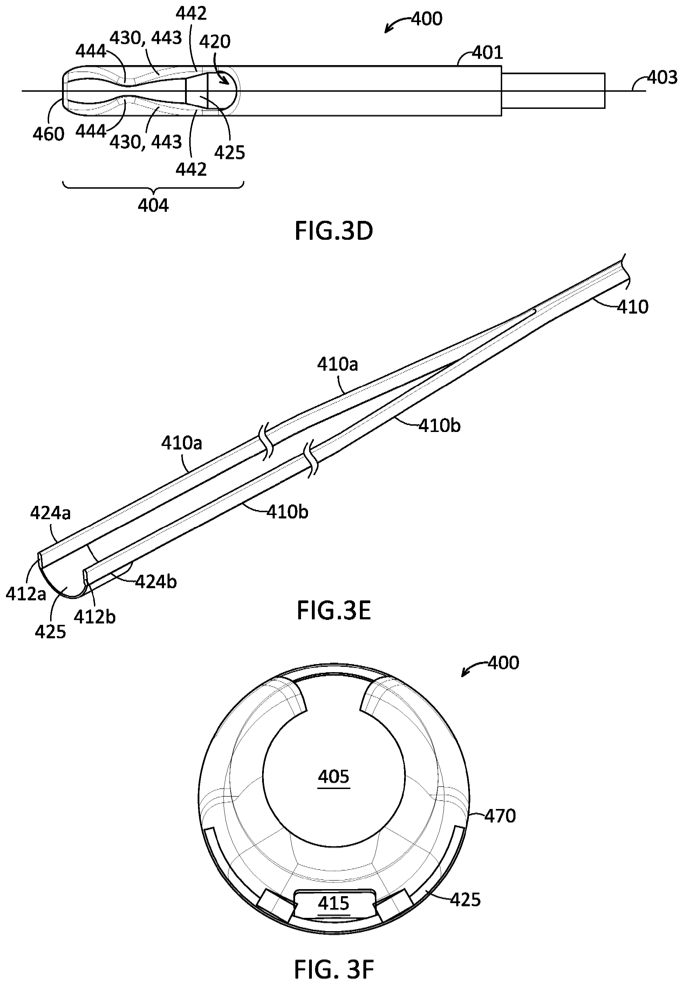

FIGS. 3A**-F** illustrate the guard member 400 according to embodiments of the inventions. The guard member 400 comprises a pull wire 410, the pull wire 410 bifurcates into two elongated portions 410a and 410b at a distal section 411 of the pull wire 410, as shown in **FIG. 3E****.** The bifurcated pull wire portions 410a and 410b may be produced by laser cutting the distal section 411 of the pull wire 410, or any other suitable techniques. The two elongated portions 410a and 410b of the pull wire 410 are attached to an arcuate radiopaque marker 425 (e.g., partial or half cylindrical configuration, non-annular), as shown in **FIG. 3E****.** The elongated portions 410a and 410b of pull wire 410 are embedded or encased within guard member 400 and include respective distal end tips 412a and 412b, each of the distal end tips 412a and 412b is coupled (e.g., attachment sections by welding or other suitable techniques) to the arcuate radiopaque marker 425 **(****FIG. 3E****);** the marker 425 is also embedded or encased within guard member 400.

As shown in FIGS. 2C**-D,** the delivery catheter 304 comprises a lumen 322 configured to receive and/or accommodate the pull wire 410. The delivery catheter 304 comprises a lubricious liner (e.g., PTFE or the like) covering the inner walls of the lumen 322 (not shown) to increase the lubricity and/or decrease friction of the pull wire 410 within the lumen 322 of the delivery catheter 304, thereby facilitating smooth proximal and/or distal movement, translation and/or actuation of guard member 400 to expose and re-cover the penetrating element 350.

Embodiments of the pull wire 410 of FIGS. 3A**-F** are configured to translate the guard member 400 proximally and/or distally relative to the delivery catheter 304 to at least partially expose or cover, respectively, the penetrating element 350. The bifurcated pull wire portions 410a and 410b are further configured to evenly transfer translation forces (e.g., pull and/or push) to the guard member 400, since the bifurcated portions 410a and 410b are coupled to substantially opposing lateral sides of guard member 400. As a result, the pull wire 410 provides smoother torque, advancement and/or retraction of the guard member 400 over the penetrating element 350 compared to a single pull wire without distal bifurcated portions 410a and 410b or two separate pull wires extending from guard member 400 to the proximal end of the delivery catheter 304. For example, the pull wire 410 with bifurcated portions 410a and 410b provides a desirable 1:1 torque response between the shaft 344a of the delivery catheter 304 and the guard member 400. The elongated portions 410a and 410b of bifurcated pull wire 410 are radially disposed within the guard member 400, thereby maintaining torque response between the guard member 400 and the shaft 344a of the delivery catheter 304 when the delivery catheter 304 is rotated. Additionally, the bifurcated pull wire portions 410 and 410b are configured to provide column strength to the delivery assembly 300 during advancement of the delivery catheter 304, avoiding and/or minimizing inadvertent proximal translation of the guard member 400 and undesirable exposure of the penetrating element 350. Further, the bifurcated pull wire portions 410a and 410b are configured to resist undesirable guard member 400 rotation around the distal portion 344 of the delivery catheter, thereby reducing the likelihood that the arcuate marker 425 may provide a false indication of delivery catheter 304 orientation in the vasculature. When the target penetration site is reached, thepull wire 410 is configured to proximally translate (e.g., pull) the guard member 400 to expose the penetrating element 350, as described and depicted in further detailed in FIGS. 4A**-B.**

Referring back to FIGS. 3A**-F,** the pull wire 410 with bifurcated portions 410a and 410b allow for a reduced diameter in the lumen of the delivery assembly, compared to having multiple pull wires for the guard member 400. Further, the bifurcated pull wire portions 410a and 410b reduce production time and cost of materials during manufacturing, since the pull wire 410 may be manufactured by laser cutting and splitting the distal section 411 of a single pull wire compared to manufacturing two separate pull wires coupled to the guard member 400 that extend between distal and proximal portions of the catheter (not shown).

The pull wire 410 can be composed of uncoated stainless steel, coated (e.g., PTFE) stainless steel or other suitable coated or uncoated materials. A PTFE or other lubricious coating, liners or the like, on pull wire 410 and/or in delivery catheter lumen 322 can increase the lubricity of the wire within the delivery catheter 304, thereby facilitating smooth proximal and distal actuation of guard 400 to expose and re-cover the penetrating element 350. While the pull wire 410 embodiment depicted in FIGS. 3A**-F,** including sections 412a and 412b, have respective rectangular profiles (e.g., flat wire configuration), other pull wire embodiments can include non-rectangular cross-sectional profiles (e.g., circular, crescent). The pull wire 410 with bifurcated portions 410a and 410b, including the arcuate radiopaque marker 425, minimizes the overall profile of the guard member 400 by having less material (e.g., flat bifurcated wire and non-annular marker) and consequentially minimizing the French size of the delivery catheter 304.

The arcuate radiopaque marker 425 can comprise platinum-iridium 90/10 alloy or other suitable materials that provide sufficient radiopacity. Further, the arcuate radiopaque marker 425 allows the connection areas 424a and 424b to be coupled to the respective elongated portions 410a and 410b of the pull wire 410, as shown in **FIG. 3E****.** The two elongated portions 410a and 410b of the pull wire 410, each comprises respective distal end tips 412a and 412b; the distal end tips 412a and 412b are coupled (e.g., attached, welded) to the connection areas 424a and 424b of the arcuate radiopaque marker 425. It should be appreciated that in embodiments where the pull wire 410 has a lubricious coating (e.g., PTFE), the distal end tips 412a and 412b do not include coating; the uncoated distal end tips 412a and 412b of the pull wire 410 allow for a weld or other attachment at the connection areas 424a and 424b of the radiopaque marker 425.

As stated above, the arcuate radiopaque marker 425 comprising partial or half cylindrical configuration **(**FIG. 3A**-B** and 3E-F) minimizes the profile of the guard member 440. Further, the arcuate marker 425 is configured to determine the location (e.g., by tracking the marker position and orientation within the guard member 400) of the concentrically disposed penetrating element 350, when alignment of the arcuate marker 425 with a radiopaque marker 354 of the penetrating element 350 **(****FIGS. 2B****,** **4A** and **6A****)** occurs. In some embodiments, the alignment of the guard marker 425 and penetrating element marker 354 allows the clinician to determine the location of the penetrating element 350 within the guard member 400 during tracking (e.g., navigation of the delivery assembly 300 through tortuous anatomy towards the target penetration site) so that the clinician may monitor or correct inadvertent withdrawal of the guard member 400 and/or deployment, orientiation, and/or trajectory of the penetrating element 350. Additionally, the arcuate marker 425 and penetrating element marker 354 are configured to improve visualization of the delivery catheter 304 under fluoroscopy, since the arcuate marker 425 allows the clinician to determine orientation of the delivery catheter 304 and penetrating element 350 relative to a target penetration site in the vasculature; as opposed to an annular marker (not shown). As shown in **FIG. 3F****,** the arcuate marker 425, embedded within the guard member 400, is adjacently disposed to an outer surface 470 of the guard member 400, such that the second lumen 415 of the guard member 400 is partially surrounded by the arcuate marker 425. In this embodiment, the arcuate marker 425 is configured to provide structural strength to the guard member 400, further avoiding and/or minimizing undesirable tear of the guard member 400 by the elongate guide member 780 translating within the second lumen 415 of the guard member 400. With improved structural strength of the guard member 400, including second lumen 415, embodiments of the delivery catheter 304 do not need a separate, dedicated lumen to receive the elongate guide member 780, for example, as disclosed in U.S. Patent No. 10,272,230. Removal of a lumen in the delivery catheter 304 for receiving an elongate guide member can streamline the delivery catheter 304 manufacturing steps and reduce the overall French profile of the delivery catheter 304 and/or delivery assembly 300. In addition, the delivery assembly 300 usability is improved because an operator is required to thread elongate guide member 780 through only one lumen (e.g., second lumen 415 of the guard member 400) before advancing delivery catheter 304 into the patient at the venous access site toward a target penetration site in the vasculature.

As better appreciated in FIG. 3F depicting a cross-sectional view of FIG. 3A, the guard member 400 comprises the first lumen 405 configured to receive and/or accommodate the penetrating element 350, and the second lumen 415 configured to receive and/or accommodate the elongate guide member 780. The guard member 400 is further configured to allow rapid exchange with the elongate guide member 780, a distal portion of elongated guide member 780 is coupled to a stent anchor or other temporary anchoring element (not shown).

Referring back to **FIG. 2B** the guard member 400 is disposed in a delivery configuration with respect to the distal portion 344 of delivery catheter 304, thus covering penetrating element 350 (also shown in **FIG. 4A****).** The penetrating element 350 is positioned within lumen 405 of the guard member 400 and aligned with the radiopaque marker 425 of the guard member 400 such that, under fluoroscopy, the distal tip of penetrating element, for example, as confirmed by penetrating element marker 354, overlies radiopaque marker 425. The guard member 400 can be approximately 0.5" (1.27 cm) long or other suitable dimensions sufficient to cover penetrating element 350 on the distal portion 344 of the delivery catheter 304. The guard member 400 and the delivery catheter 304 including the penetrating element 350 are slidable relative to each other. For example, the guard lumen 405 is sized to allow guard 400 to retract proximally over the penetrating element 350 and the distal portion 344 of the delivery catheter 304. For example, the inner diameter of guard lumen 405 can be approximately 0.0385" (0.09779 cm).

As depicted in FIG. 3A**-B,** the marker 425 and the pull wire bifurcated portions 410a and 410b are embedded or encapsulated within the wall of guard member 400. Guard member 400 is composed of polymeric materials such as polyether block amide (Pebax^{®} available from Arkema Group), HTPE, PTFE, Nylon, polyurethane, urethanes or any other polymeric material. Pebax^{®} embodiments of guard 400 can range from 27D to 70D hardness (e.g., Pebax^{®} 63D). The wall thickness of guard 400 can vary depending on top-to-bottom orientation of the guard.

As illustrated in the side view of the guard member 400 in **FIG. 3A****,** the guard member 400 comprises a distal portion 404 having a contour/profile similar to a one period sine function with a peak 444 (e.g., maximum) and a valley 442 (e.g., minimum). It should be appreciated that the opposite side view (not shown) of the distal portion 404 of the guard member 400 of **FIG. 3A** comprises a contour/profile with a mirror-image one period sine function. The contour/profile in distal portion 404 of guard member 400 can facilitate delivery catheter 304 access to tortuous anatomy where the distal portion 404 of guard member 400 first accesses and advances through a narrowing or sharp bend, thereafter allowing the distal portion 344 of the delivery catheter 304 to track and follow through the narrowing or bend.

As shown in the perspective views **(**FIGS. 3B**-C)** and the top view **(****FIG. 3D****)** of the guard member 400, the guard member 400 comprises a tubular structure 401 having an aperture 420 at the distal portion 404 of the guard 400. The distal portion 404 of guard member 400 defines the aperture 420 by having the respective peaks 444 of the sine functions to gradually approach each other, and the respective valleys 442 of the sine functions to gradually separate from each other. The split shape of the distal portion 404 of guard member 400 and convergence of the aperture 420 transitioning from valleys 442 to peaks 444 of a deflecting element 430 (better appreciated in FIGS. 3C**-D)** may be formed by laser cut of the tubular structure 401, molding or any other suitable technique. The shape of the distal portion 404 of guard member 400 creates an atraumatic tip 460 and further facilitates access to narrow or tortuous vasculature as the clinician navigates the delivery assembly toward the target penetration site. For example, the IPS diameter at the ostium in the internal jugular vein is often smaller than the IPS diameter at a target penetration site in the vessel (e.g., axial level of the jugular tubercle) and the operator must track delivery catheter 304 through the ostium to target penetration site for accessing the subarachnoid space. The contour/profile in distal portion 404 of guard member 400 initiates access of distal portion 344 of the delivery catheter 304 through the ostium into the vessel, which allows the remainder of the device to advance distally toward the target penetration site.

Further, the split shape of the distal portion 404 of guard member 400 comprises arcuate slopes 443 between the valleys 442 and the peaks 444, where the arcuate slopes 443 **(****FIG. 3B****)** form the deflecting element 430 configured to deflect the penetrating element 350 away from the elongate guide member 780 **(****FIG. 4B****)** and toward the penetration site. In some embodiments, the deflecting element 430 may be formed with steeper, or less steep, slopes 443 by varying the height of the peaks 444, depth of the valleys 442 and/or the angle "θ" **(****FIG. 3A****)** between deflecting element 430 and the longitudinal axis 403 parallel to the lumen 415 of the guard member 400 **(**FIGS. 3A**-C).** The angle "θ" of the slopes 443 that form the deflecting element 430 may range from about five degrees to about 40 degrees, or more. Increasing the angle "θ" of the slopes 443 relative to the longitudinal axis 403 of the guard member 400, increases the distance or separation between the penetrating element 350 and guide member 780, thus increasing the angle of penetration of penetrating element 350 with respect to the target site. In turn, greater separation between the penetrating element 350 and guide member 780 improves penetrating element 350 engagement with IPS wall 114 to ensure that the penetrating element 350 accesses the SAS, and can be particularly advantageous in vascular anatomies that do no exhibit sufficient curvature about a target penetration site to enable penetrating element 350 to travel off-axis from the guide member 780 and access the SAS.

FIGS. 4A**-B** illustrate an interface between the penetrating element 350 and guard member 400, according to embodiments of the invention. The guard member 400 is disposed over penetrating element 350 **(****FIG. 4A****)** to guard against inadvertent punctures in the vasculature while tracking the delivery catheter to the target penetration site. The guard member 400 may translate proximally over the distal portion of the delivery catheter 304 and the penetrating element 350 (shown by arrow I). Additionally, the penetrating element 350 may translate distally towards the deflecting element 430 of the guard member 400 (shown by arrow II) to expose the penetrating element 350 at the target penetration site **(**FIGS. 4A**-B** For example, the clinician may retract the guard member 400 proximally, so that the penetrating element 350 disposed at the distal portion of the delivery catheter 304 contacts the deflecting element 430 / slopes 443 of the guard member 400, thus deflecting the penetrating element 350 away from the longitudinal axis 403 of the guard member 400 and elongate guide member 780 **(****FIG. 4B****).** In alternate embodiments of the delivery catheter 304, the clinician may advance the penetrating element 350 distally to contact the deflecting element 430 / slopes 443 of the guard member 400.

FIGS. 5A**-C** illustrate another view of the interface between the penetrating element 350 and guard member 400, according to embodiments of the invention. For example, FIGS. 5A**-C** show the interface between the penetrating element 350 and guard member 400 after the guard member 400 has been withdrawn proximally to expose the penetrating element 350 and/or the penetrating element 350 has been advanced distally to contact the deflecting element 430 / slopes 443 of guard member 400. The split shape of the distal portion 404 of guard member 400 having the slopes 443 is configured to allow the deflecting element 430 to open up and collapse around the shaft 344a of the delivery catheter 304 when the penetrating element 350 of the delivery catheter is retracted into the guide catheter 320. FIGS. 5A**-C** depict a sequential down-the-barrel view where the delivery catheter 304 is retracted or withdrawn into guide catheter 320 (e.g., while withdrawing the delivery catheter 304 from the SAS) and the split shape of the deflecting element 430 opens up and allows for the penetrating element 350 to at least partially fit within the aperture 420, substantially maintaining the diameter of the guide catheter 320. In contrast, in embodiments where the deflecting element is a solid feature of guard member 400 (e.g., without splits, respective deflecting element 430 or slopes 443), when the delivery catheter 304 is retracted or withdrawn, the penetrating element 350 will be disposed adjacent (e.g., wedging) to a solid deflecting element of the guard member (e.g., double barrel) thus undesirably extending the diameter of the guide catheter (not shown); this undesirable guide catheter extension can cause inadvertent shunt 200 removal from the SAS if, prior to shunt deployment, the shroud 800 and shunt 200 are also wedged in the guide catheter opening by a solid deflecting element of the guard member.

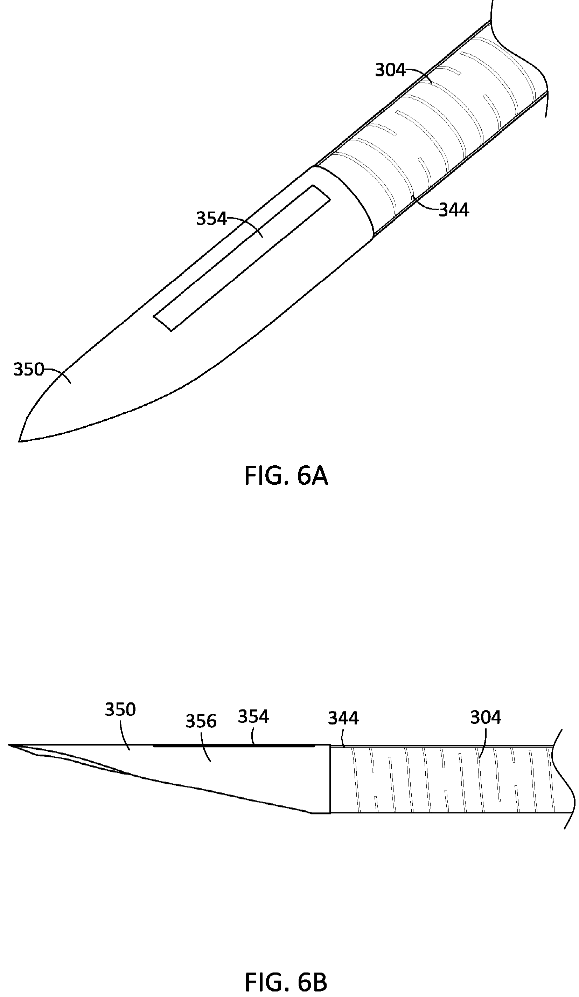

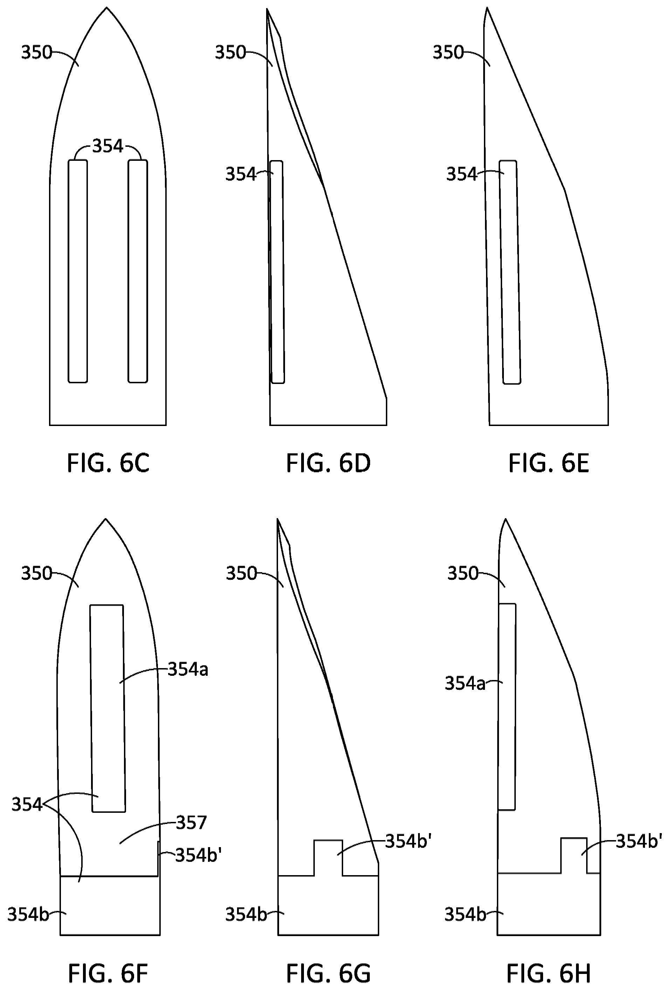

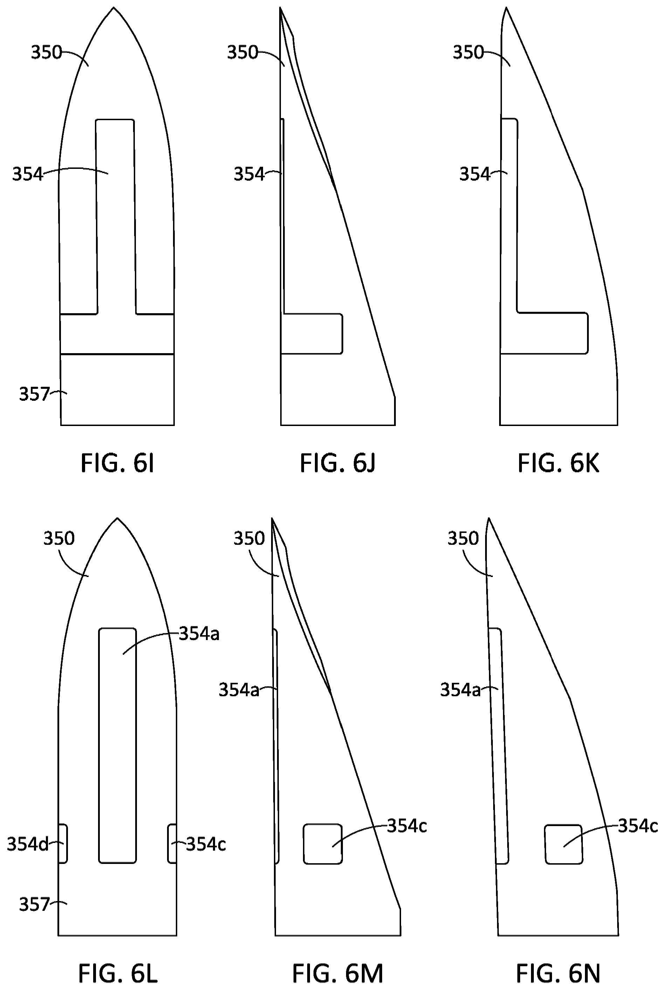

FIGS. 6A**-N** illustrate the penetrating element 350, according to various embodiments of the inventions. The penetrating element 350 comprises a sharp tapered, cannula-like end, bevel, pencil, or quincke tip needle, or the like. **FIGS. 6A-****B** illustrate the penetrating element 350 disposed at the distal portion 344 of the delivery catheter 304. The penetrating element 350 further comprises a radiopaque marker 354 embedded therein, as better shown in **FIG. 6A****.** FIGS. 6C**-N** illustrate alternative embodiments of the radiopaque marker 354 embedded in the penetrating element 350. The embedded marker 354 does not protrude from the outer surface 356 of penetrating element 350 **(**FIGS. 6A**-N** or from the inner surface of the of penetrating element 350 (not shown), allowing smooth inner 356 and outer surfaces of the penetrating element 350. The radiopaque marker 354 may be embedded in a recess (not shown) of the outer surface 356 of penetrating element 350 and attached by welding or other suitable techniques. The radiopaque marker 354 may comprise platinum-iridium 90/10 alloy or other suitable materials that provide sufficient radiopacity. The radiopaque marker 354 is configured to allow the clinician to determine the location of the penetrating element 350 relative to an anatomic landmark (e.g., IPS 102, CP angle cistern), and further allow alignment with radiopaque marker 425 of the guard member 400, as shown in **FIG. 2B****.** Improved visibility of the penetrating element 350 through the radiopaque marker 354 further mitigates the risks of the shunt being deployed in the subdural space or venous system and further, mitigating or avoiding the risks of injury to critical structures in the SAS by penetrating element 350 (e.g., brainstem, vertebral artery, basilar artery, anterior inferior cerebellar artery, cranial nerve VI (abducens nerve)). The radiopaque marker 354 facilitates visualization of the penetrating element 350 preventing inadvertent deployment of the penetrating element 350 through the guard member 400 when the clinician navigates the delivery assembly 300 through tortuous anatomy towards the target penetration site.

The radiopaque marker 354 may comprise an elongated configuration (e.g., longitudinally aligned with the tip of the penetrating element 350), as shown in FIGS. 6A**-B.** Alternatively, the radiopaque marker 354 may comprise a set of at least two elongated markers disposed substantially parallel to each other (e.g., off-set with respect to the tip of the penetrating element 350), as shown in **FIG. 6C****.** Although FIGS. 6D**-E** show a single elongated marker 354, FIGS. 6D**-E** are side views of **FIG. 6C** having a set of two markers. FIGS. 6F**-H** illustrate another alternative embodiment of the radiopaque marker 354 of the penetrating element 350, where the radiopaque marker 354 comprises an elongated marker 354a and a annular marker 354b. The elongated marker 354a can be longitudinally aligned with the tip of the penetrating element 350, similar to **FIG. 6A****,** while the annular marker 354b is disposed at a proximal portion 357 of the penetrating element 350. The annular marker 354b comprises a side portion 354b' (e.g., square, rectangle or other suitable shape) as shown in **FIGS. 6F-6H****.** **FIGS. 6I-6K** illustrate yet another embodiment of the radiopaque marker 354 of the penetrating element 350, where the radiopaque marker 354 comprises a "T" shape. The "T" shape of the marker 354 has the base of the "T" longitudinally aligned with the tip of the penetrating element 350, and the top of the "T" disposed in a curved, semi-anular configuration at the proximal portion 357 of the penetrating element 350, as shown in **FIGS. 6I-6K. FIGS. 6L-6N** illustrate another alternative embodiment of the radiopaque marker 354 of the penetrating element 350, having an elongated marker 354a, similar to **FIG. 6F****,** and two side markers 354c and 354d disposed at the proximal portion 357 of the penetrating element 350.

The embodiments of the radiopaque marker 354 shown in **FIGS. 6A-6N** are configured to provide visual feedback regarding the penetrating element 350 orientation in the vasculature, which facilitates visualization and prevents inadvertent deployment of the penetrating element 350 through the guard member 400. The various embodiments of the radiopaque marker 354 facilitate visualization of the penetrating element 350 about a target penetration site, and visualization of the trajectory of penetrating element 350 into the SAS. For example, the marker 354b and portion 354b' **(**FIGS. 6F**-H),** the top "T" shape of marker 354 **(**FIGS. 6I**-K),** and markers 354c and 354d **(**FIGS. 6L**-N)** may allow the clinician to determine the location of the penetrating element 350 relative to the radiopaque marker 425 of the guard member 400, while allowing the clinician to determine the orientation of the penetrating element 350 within the guard member 400 and in the vasculature.

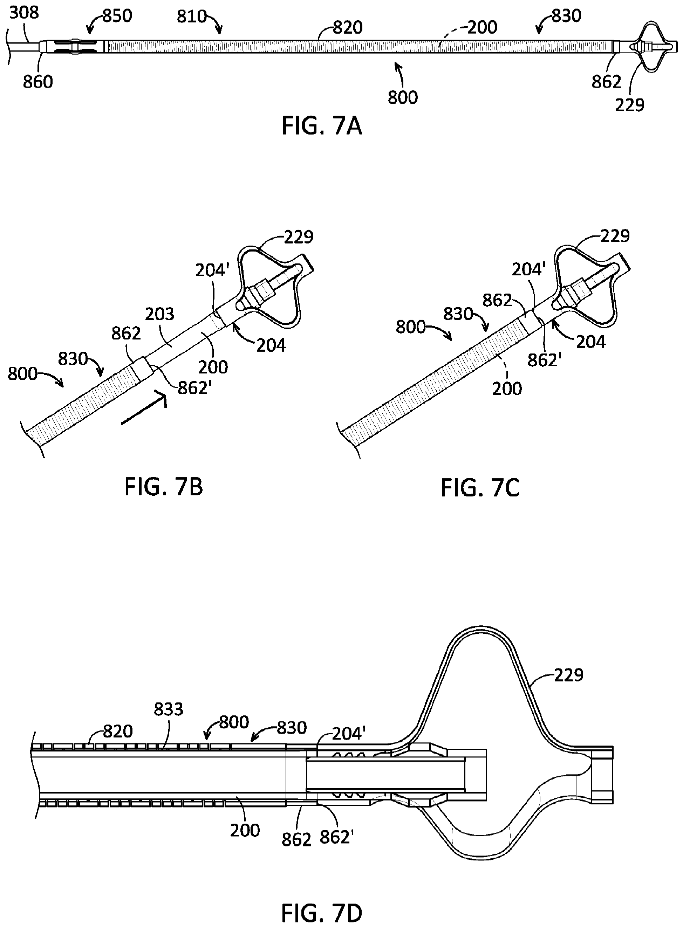

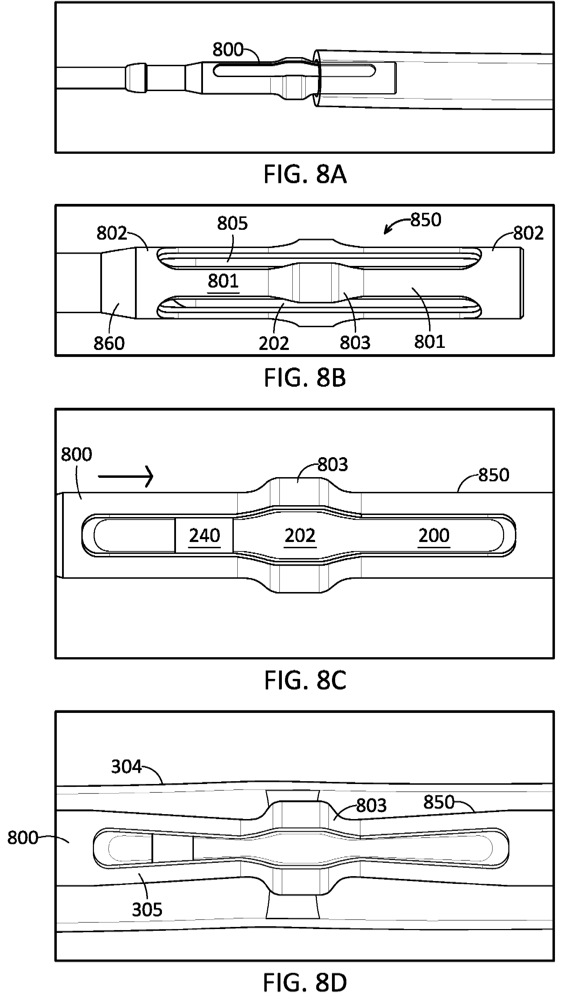

FIGS. 7A**-D** and FIGS. 8A**-D** illustrate the implant (e.g., shunt) and the shroud according to the embodiments of the inventions. **FIG. 7A** shows the combined shunt 200 and shroud 800, FIGS. 7B**-D** show the interface between the distal portion of shroud 800 and the distal portion of the shunt 200, while FIGS. 8A**-D** show the interface between the proximal portion of shunt 200 and the proximal portion of shroud 800 and a shroud proximal retention feature 850. The shroud 800 is configured to be at least partially disposed over and movable relative to the shunt 200 **(****FIGS. 7B-C).** The shroud 800 is configured to minimize friction on and/or stretching of the shunt 200 to avoid or mitigate the risk of failure (e.g., tearing of the polymeric shunt body, removal of heparin or other coating on the shunt body, damage to the shunt valve, or the like) of the shunt 200. In addition, the disclosed embodiments of the shroud 800 provide a smoother and more controlled release of the shunt 200 because the shroud 800 does not capture a stretched, "necked down" (e.g., temporarily reduced outer diameter due to shunt body stretching and elongating) compared to shroud embodiments (e.g., shunt delivery shuttle embodiments disclosed in U.S. Patent App. Pub. No. US20200030588A1) that encapsulate a stretched, elongated shunt 200.

The shroud 800 comprises a tubular body 820 having a proximal portion 810, a distal portion 830, and a lumen 833 extending therebetween, as shown in FIGS. 7A**-D.** The shroud 800 further comprises a proximal marker 860 and a distal marker 862, as shown in **FIG. 7A****.** The proximal and distal markers 860 and 862 are composed of tantalum, or any other radiopaque material, suitable for visualization of the shroud 800 under fluoroscopy. Particularly, the proximal and distal markers 860 and 862 are configured to allow visualization of the shroud 800 relative to the shunt 200 during advancement, deployment and/or delivery of the shunt 200 at the target site. The shroud distal marker 862 comprises an annular configuration, while the shroud proximal marker 860 comprises an annular tapered configuration **(****FIG. 7A****)** for gradually leading the shroud 800 (e.g., when the shroud 800 translates from within the penetrating element 350 to advance the shunt 200 into the target site). In alternative embodiments, the shroud proximal and distal markers 860 and 862 may comprise any suitable configuration.

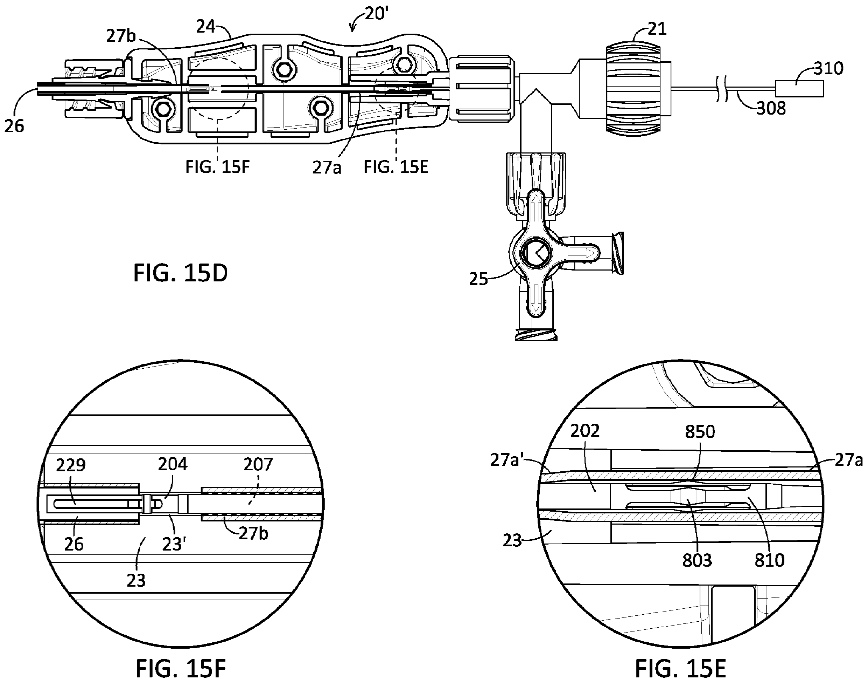

In **FIG. 7A****,** the delivery wire 308 is shown proximately to the proximal marker 860 of the shroud 800. The delivery wire 308 is configured to orient, advance and/or retract the shroud 800 and shunt 200 through the first lumen 305 of the delivery catheter304 (not shown). The delivery wire 308 comprises a suitable dimension (e.g., diameter of approximately 0.010" (0.254 mm) to 0.018" (0.4572 mm)) to navigate the shroud 800 and shunt 200 through the first lumen 305 of the delivery catheter 304 into the SAS. As known in the art, the delivery wire 308 comprises a tapered configuration for gradual stiffness (i.e., proximal section) to flexible (i.e., distal section) transitions, such that the delivery wire 308 transfers translational and rotational forces to orient, advance and/or retract the shroud 800 and shunt 200 including when distal portion 344 of delivery catheter 304 is disposed within tortuous anatomy. In some embodiments, the delivery wire 308 is further tapered along the length the shroud 800 to allow for smooth actuation of the shunt 200. Embodiments of the delivery wire can include a stop 310 (e.g., a bushing as shown in **FIG. 15D****)** to prevent the operator from advancing the delivery wire too far distally and losing the proximal end of the delivery wire inside of the transfer device tuohy borst during the procedure.

The tubular body 820 of the shroud 800 comprises super elastic Nitinol (or any other suitable super-elastic alloys or materials) having an interrupted spiral cut pattern **(**FIGS. 7A**-D.** or any other suitable cut pattern configured to provide flexibility and kink resistance to the shroud 800 during tracking through first lumen 305. In some embodiments, the shroud 800 includes at least one axial spine along the length of the tubular body 820. For example, the cut pattern of tubular body 820 includes an interrupted spiral cut leaving sequential uncut portions of the tubular body 820 forming an axial spine (not shown). In the embodiments of the shroud 800 having the at least one axial spine, the axial spine is configured to provide column strength to the shroud 800 to assist the shroud 800 with the translation of the shunt 200 through first lumen 305 of the delivery catheter 304. Additionally, the axial spine of the shroud 800 avoids or minimizes spring forces on the tubular body 820, such as when the shroud 800 transitions from being disposed within the delivery catheter 304 to being deployed out of the penetrating element 350; this advantageously prevents distal portion 830 of shroud 800 and/or shunt 200 from springing or "jumping" at the target deployment site (e.g., excessive and inadvertent distal movement of shunt 200) as the shroud 800 and shunt 200 are advanced through, free and clear of the penetrating element 350. Further, the cut pattern of the shroud 800 having the at least one axial spine is still configured to provide flexibility and kink resistance during tracking of the shroud 800 within the first lumen 305 of the delivery catheter 304. The embodiments of the disclosed inventions are an improvement, compared to shunt delivery shuttle embodiments disclosed in United States Patent App. Pub. No. US20200030588A1. Embodiments of shroud 800 prevent instances where the shunt cannot be released from the delivery assembly because the shroud resists potential kinking and crimping of the tubular body and lumen on shunt body that could prevent shunt from fully releasing from shroud.

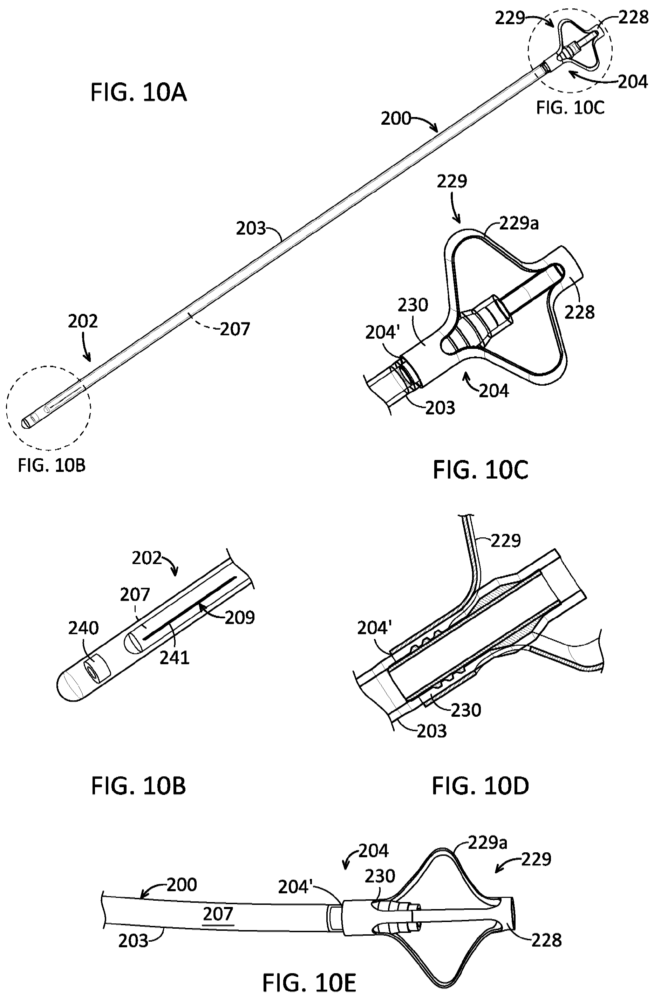

The shroud 800 is configured to slide over the shunt 200 and distally engage the shunt **(**FIGS. 7B**-C)** to translate and advance the shunt 200 through the first lumen 305 and penetrating element 350 of the delivery catheter 304 towards the target implantation site. Further, the shroud 800 is configured to engage (e.g., contact, abut, grasp) a proximal portion 202 of the shunt 200 **(**FIGS. 8C**-E)** assisting with the controlled positioning and advancement (e.g., moving, translating, pushing) of the shunt 200 through the delivery assembly 300. **FIG. 7B** illustrates the shroud distal portion 830 sliding over an elongated body 203 of the shunt 200 and **FIG. 7C** illustrates the shroud 800 disposed over the shunt 200 towards an anchoring mechanism 229 of the shunt (e.g., malecot). As shown in **FIGS. 7B** and 10D**-E,** the shunt body 203 comprises an outer diameter "OD" smaller than the OD of the shroud 800, such that the shunt 200 is slidably disposed within the lumen 833 of the shroud 800, except that the OD of the anchoring mechanism 229 of the shunt 200 is substantially the same OD as the OD of the shroud 800. Further, the anchoring mechanism 229 of the shunt 200 comprises an annular edge 204' configured to contact/abut an annular edge 862' of the shroud 800 marker 862 **(**FIGS. 7B**-D** and **10D-E).** During the engagement of the shroud 800 with the shunt 200, while the shroud 800 advances over the shunt 200, the annular edge 862' of the shroud 800 marker 862 contacts/abuts the annular edge 204' of the anchoring mechanism 229 at the distal portion 204 of the shunt 200 **(****FIGS. 7B** and **10E****).** As better appreciated in FIGS. 7C**-D,** when the annular edge 862' of the marker 862 is in contact with the annular edge 204' of the anchoring mechanism 229 of the shunt 200, the OD of the engaged shroud 800 and shunt 200 is substantially the same, such as, the OD is flush, substantially flush, or even between the shroud distal portion 830 (e.g., distal marker 862) and the distal anchoring mechanism 229 of the shunt 200. Therefore, the distal portion 830 of the shroud 800, when engaged to the distal portion 204 of the shunt 200 at the distal anchoring mechanism 229, is configured to translate, advance, and/or push the shunt 200 through the delivery catheter 304 first lumen 305 and the penetrating element 350 for deployment and delivery in the target site.

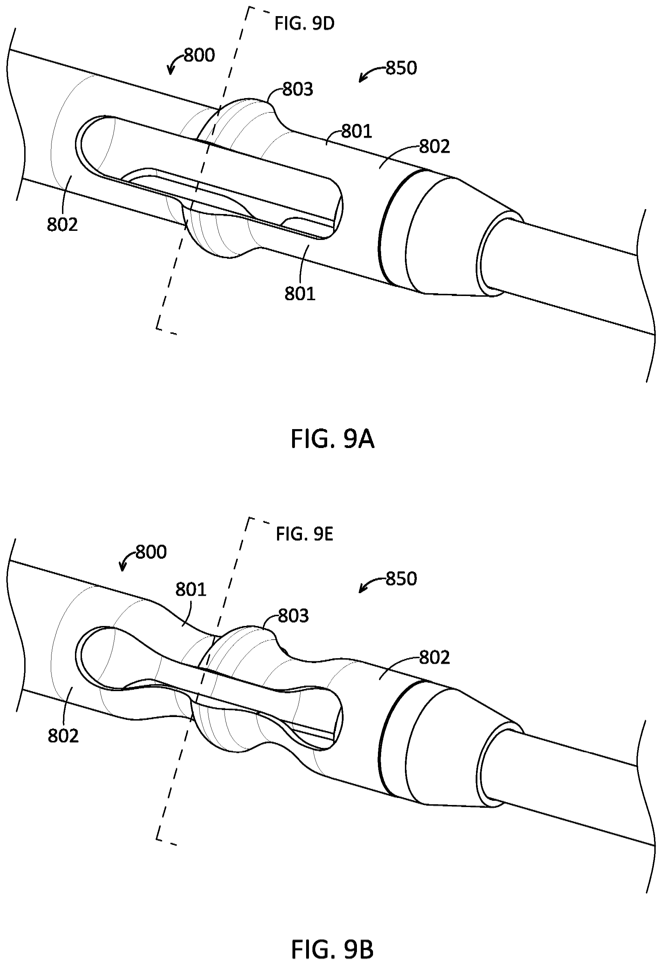

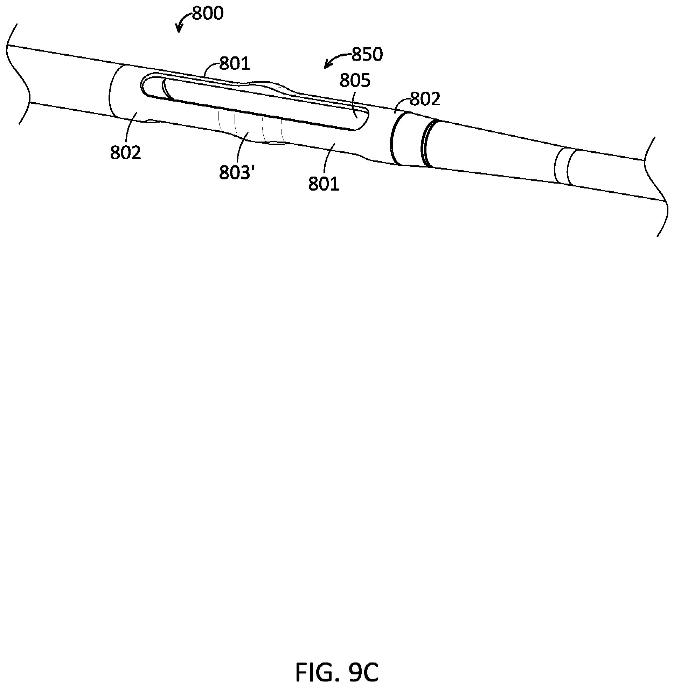

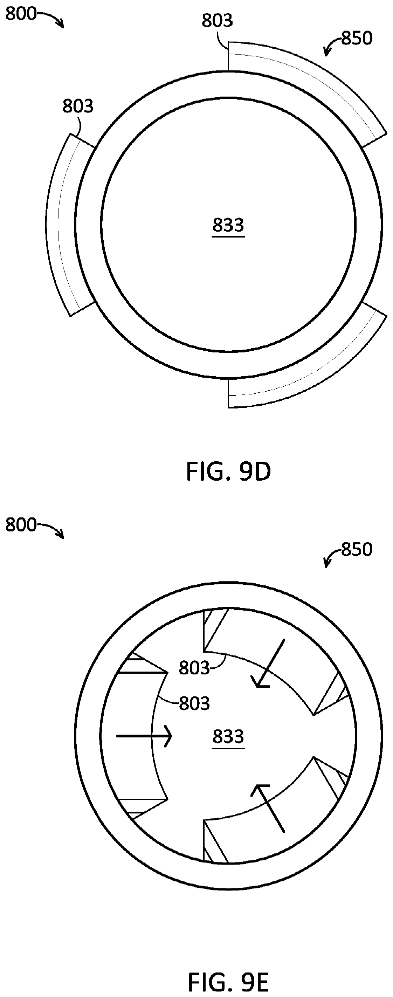

FIGS. 8A**-F** depict the actuation of embodiments of the shroud 800 with the proximal portion 202 shunt 200. The proximal portion 810 of the shroud 800 comprises a proximal retention feature 850 **(**FIGS. 7A**, 8B-F** and **9A-B).** FIGS. 9A**-E** illustrate alternative embodiments of a proximal retention feature 850 of the shroud 800 in a relaxed/expanded configuration **(****FIG. 9A****,** **9C** and **9D****)** and in a collapsed/contracted configuration **(****FIG. 9B** and **9E****).** The proximal retention feature 850 is configured to hold, grasp and/or restrain the proximal portion 202 of shunt 200 to assist with the advancement of the shunt 200, for example, through the delivery catheter lumen 305 toward the target site. The proximal retention feature 850 is configured to slide over the proximal portion 202 of shunt 200 and the proximal portion 202 of shunt 200 is configured to be disposed within the retention feature 850 **(****FIG. 8B****),** such that the retention feature 850 grasps the proximal portion 202 of shunt 200 by assuming the retention feature 850 collapsed/contracted configuration **(**FIGS. 8D-E, 9B and **9E)** (e.g., when the proximal portion 202 of shunt 200 and shroud retention feature 850 are constrained within delivery catheter first lumen 305). The proximal retention feature 850 comprises a plurality of elongated members 801 extending between two annular members 802, each of the plurality of elongated members 801 comprises a protrusion 803 disposed approximately in the middle of the respective elongated members 801, as better appreciated in **FIGS. 8B** and 9A**-C.**

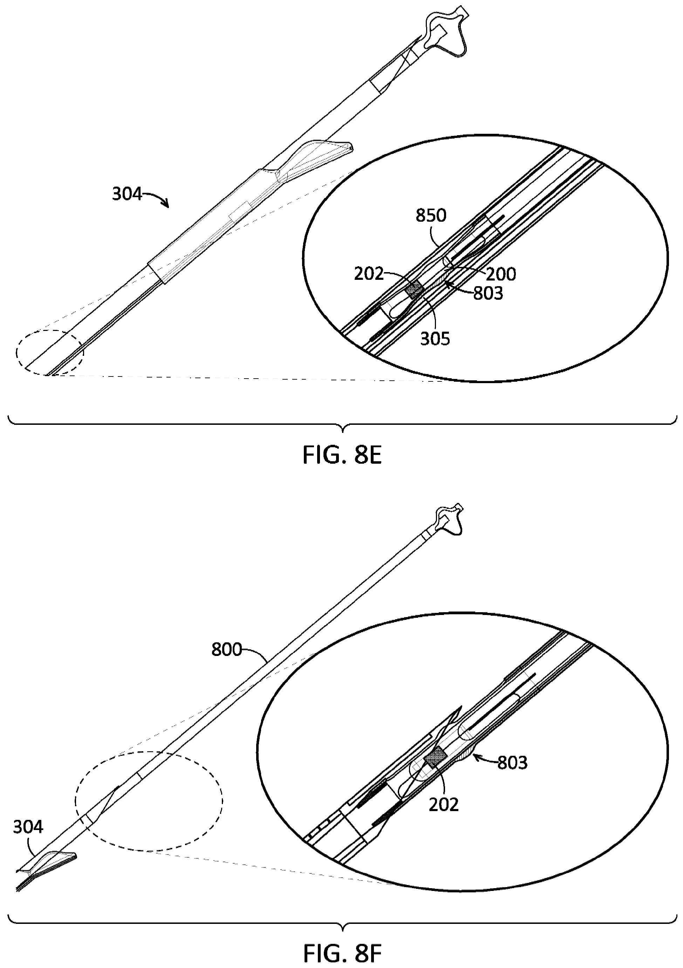

**FIG. 8E** illustrates a detailed inside view of the delivery catheter 304, where the constrained retention feature 850 of the shroud 800 is engaged with shunt proximal portion 202 within the delivery catheter lumen 305. The proximal portion 202 of the shunt 200 is securely restrained by the elongated members urged toward the shunt by the constrained protrusions 803 of retention feature 850. While **FIG. 8F** illustrates a detailed view of the shunt proximal portion 202 and proximal retention feature 850 emerging from the delivery catheter 304, where the protrusions 803 of the retention feature 850 are unconstrained from the shunt proximal portion 202 upon deployment from the delivery catheter penetrating element 350.

**FIG. 9C** illustrates an alternative embodiment of FIGS. 9A**-B,** where the protrusion 803' of **FIG. 9C** comprises a smaller radius, thickness and/or surface area (as further illustrated in FIGS. 8E**-F)** than the protrusion 803 of FIGS. 9A**-B.** Variations on the radius, thickness and/or surface of the protrusion 803 of the retention feature 850 of the shroud 800 depends on the desired retention (e.g., compression force exerted to the shunt or other device) needed on the shunt 200 disposed within delivery catheter 304 first lumen 305. It should be appreciated that a larger radius, thickness and/or surface of the protrusion 803 will exert greater compression force on the shunt 200, when the shunt 200 is disposed within the shroud lumen 833, and the shunt 200 and shroud 800 are disposed within delivery catheter 304 first lumen 305 **(****FIG. 8D****).**

In further alternative embodiments, one or more protrusions 803 may be disposed along any suitable portion of their respective elongated members 801 of the proximal retention feature 850. The protrusions 803 of the proximal retention feature 850 are configured to gradually lead the shroud 800, when the shroud 800 translates within the delivery catheter first lumen 305 and through the penetrating element 350 to advance the shunt 200 into the target site. Additionally, the proximal retention feature 850 comprising the plurality of elongated members 801 have less material (e.g., slots 805 between the elongated members 801 in **FIG. 8B****)** as opposed to a cylindrical retention feature with constant surface area (not shown). The proximal retention feature 850 having less material or surface area is configured to minimize friction between the shroud 800 and the penetrating element 350, which facilitates advancement of the shroud 800 through the delivery catheter lumen 305 when the catheter is disposed within tortuous anatomy.

The shroud 800 may be composed of titanium, tantalum, stainless steel, Nitinol, or other super-elastic alloys or materials. In some embodiments, the shroud 800 may be manufactured by cutting, electropolishing, and/or welding suitable materials and components to produce the shroud 800. For example, the retention feature 850 may be manufactured by laser cutting a tubular member, or by welding the plurality of elongated members 801 into the two annular members 802.

FIGS. 9D**-E** illustrate cross-section views of the proximal retention feature 850 of **FIGS. 8F** and **8E****,** respectively. As shown in **FIG. 9D****,** the protrusions 803 of retention feature 850 of the shroud 800 are in a relaxed, expanded or unconstrained configuration, and the lumen 833 of the shroud 800 is unobstructed by the protrusions 803. In the unconstrained configuration, the protrusions 803 will not grasp or compress the shunt proximal portion and the shunt, which will be freely movable within the shroud lumen 833. The unconstrained configuration facilitates loading the shunt into the shroud 800, for example, during device packaging and sterilization, and deployment of the shunt at the target location **(****FIG. 8F****).** As shown in **FIG. 9D****,** the protrusions 803 of retention feature 850 of the shroud 800 are in a collapsed, contracted or constrained configuration. When the protrusions 803 are constrained (e.g., by advancing the shroud 800 and shunt 200 distally through the transfer device lumen 26 and into the delivery catheter lumen 305), the protrusions 803 are biased into the shroud lumen 833. When constrained, the protrusions 803 grasp or compress onto the shunt proximal portion 202 **(**FIGS. 8D**-E),** preferably proximal to the shunt valve 209, which occurs when the shunt 200 and shroud 800 are advanced through the transfer device lumen 26, through handle 10, and into the first lumen 305 of delivery catheter 304. As shown by the arrows of **FIG. 9E****,** the protrusions 803 are biased to be constrained radially inward when proximal retention feature 850 is constrained within the delivery catheter lumen 805. The retention feature 850 constrained configuration within the delivery catheter lumen 805 maintains positive shroud 800 engagement with the shunt 200 **(**FIGS. 8D**-E),** which enables the delivery wire 803 to advance and retract the shunt 200 and shroud 800 within the delivery catheter lumen 305. When the shroud 800 and shunt 200 are advanced distally via delivery wire 308 through delivery catheter lumen 305 and out of the penetrating element 350 for shunt deployment, the retention feature protrusions 803 are no longer constrained by the inner diameter of delivery catheter lumen 305; the retention feature elongated members 801 expand radially to release retention feature 850 engagement with shunt proximal portion 202 **(****FIG. 8F****).** FIGS. 10A**-E** illustrate perspective, detailed and cross-sectional views of the shunt 200 (e.g., shunt), according to embodiments of the disclosed inventions. The shunt 200 comprises the proximal portion 202, a body portion 203, the distal portion 204 and a lumen 207 extending therebetween, as shown in **FIG. 10A****.** As shown in the detailed view of **FIG. 10B****,** the proximal portion 202 of the shunt 200 comprises a valve 209 and a radiopaque marker 240. The valve 209 is configured to be in fluid communication with the shunt lumen 207. In some embodiments, the valve 209 is a one-way valve comprising one or more slits 241. The marker 240 comprises a cylindrical band embedded in the proximal portion 202 of the shunt 200 **(****FIG. 8C** and **10B****).** To improve engagement with proximal retention feature 850 and minimize impact to the valve 209 and CSF lumen 207 of shunt 200, the proximal tip of shunt 200 (e.g., the shunt proximal portion 202 proximal to the proximal most edge of shunt lumen 207, shown in **FIG. 10B****)** can be a solid or substantially solid piece of polymeric material of shunt body 203 and the proximal marker 240. In alternative embodiments, the marker 240 may be swaged over the distal portion 204 of the shunt 200. The marker 240 is composed of any suitable radiopaque material and comprises a suitable surface area to assist visualization of the shunt 200 under fluoroscopy, avoiding or minimizing risks to patient during the accessing of the CP angle cistern.

The shunt 200 (e.g., proximal 202 and body 203 portions) includes elastomeric polymer(s) suitable for implant applications including, but not limited to, silicone, polyurethane, polycarbonate urethane, thermoplastic polyurethane, aromatic or aliphatic polycarbonate thermoplastic polyurethane, silicone/polyurethane blends (e.g., thermoplastic silicone polycarbonate polyurethane comprising 20% silicone copolymer), or polyurethane silicone blends (e.g., polyurethane silicone copolymer). The shunt 200 materials are selected to advantageously resists thrombus formation, particularly on the proximal portion 202 of the shunt 200. Optionally, the shunt 200 includes an anti-thrombotic coating to prevent thrombus formation including, but not limited to, heparin-based or phosphorylcholine-based anti-thrombotic coatings.

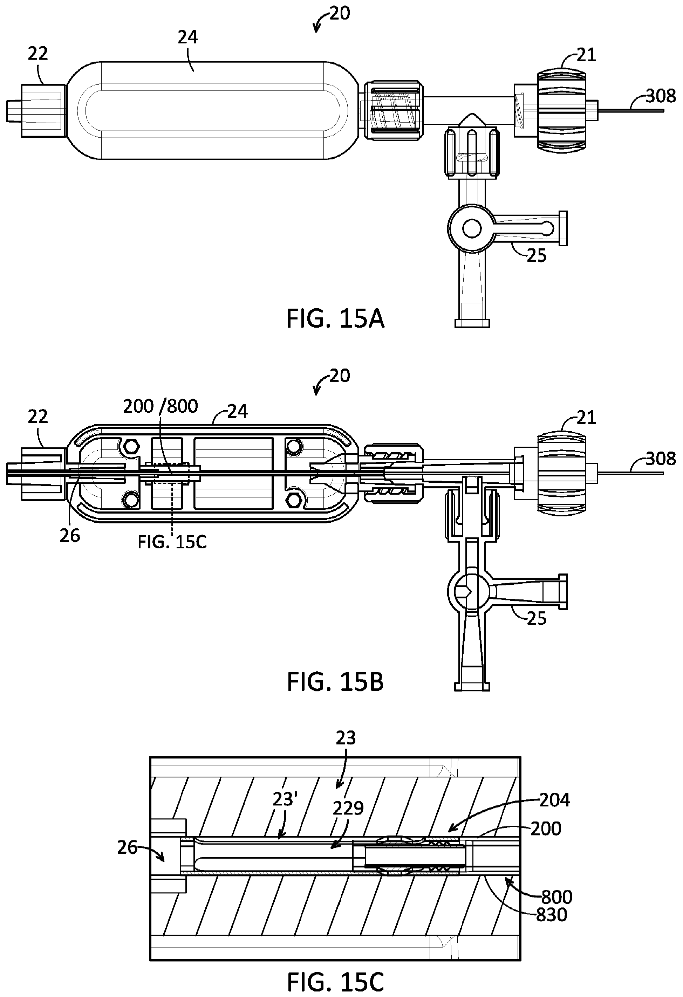

The distal portion 204 of the shunt 200 comprises the anchoring mechanism 229 (e.g., malecot) having a compressed configuration **(****FIG. 15C****)** for navigating through the delivery catheter 304 and an expanded configuration **(****FIGS. 7A**-D, 10A, and **10C-E)** for anchoring the shunt 200 at the target site in the patient. The anchoring mechanism 229 is composed of titanium, stainless steel, Nitinol, or other super-elastic alloys. The anchoring mechanism 229 comprises a proximal retainer element or collar 230, a distal radiopaque marker 228, and a plurality of elongated deformable elements 229a (e.g., arms) disposed therebetween **(**FIGS. 10A, and 10C**-E).** The proximal retainer element or collar 230 includes the distal anchoring mechanism 229, shunt distal portion 204 and annular edge 204' described above in connection with **FIGS. 7B** and **10D-E.** The proximal collar 230 and the distal marker 228 are composed of suitable radiopaque materials and can comprise an annular configuration. The deformable elements 229a are disposed radially outward in the expanded configuration of the anchoring mechanism 229. When shunt 200 is deployed out of the delivery catheter 304, the anchoring mechanism 229 transitions (e.g., self-expands) from the compressed configuration within the delivery catheter 304 to its expanded or deployed configuration. During deployment, under fluoroscopy, the clinician may observe the proximal collar 230 and the distal marker 228 move closer together, confirming that the anchoring mechanism 229 has properly transitioned to its the expanded or deployed configuration.

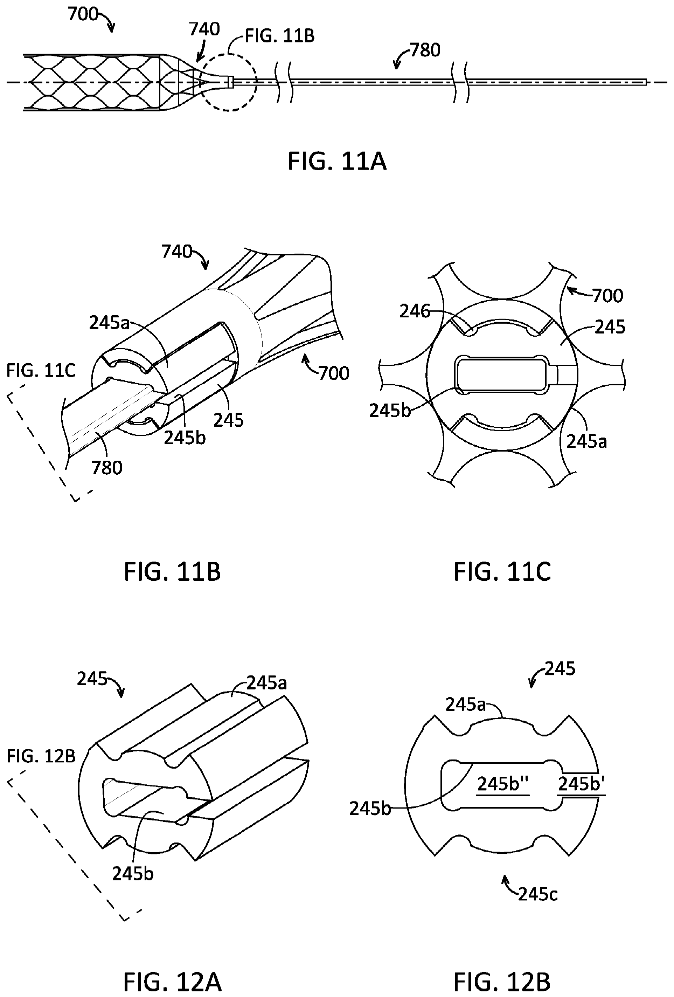

FIGS. 11A**-C** illustrate perspective and down-the-barrel views of the elongate guide member 780, an anchor 700 and a proximal marker 245, according to embodiments of the disclosed inventions. The elongate guide member 780 can have a flat, rectangular, or otherwise non-circular, cross-sectional profile, as shown for example in **FIG. 2A** and **FIG. 11B****.** As shown in the detailed view of **FIG. 11B****,** the elongate guide member 780 is coupled (e.g., directly or indirectly, attached, secured, joined, or the like) to the proximal portion 740 of the anchor 700. The anchor 700 may be composed of shape memory, self-expandable and biocompatible materials, such as Nitinol^{®}, or other super-elastic alloys, stainless steel, or cobalt chromium, or combinations thereof, and comprises a stent-like configuration. In some embodiments, the anchor 700 may include materials that are compatible with magnetic resonance imaging and have radiopacity sufficient to allow the use of known imaging techniques. Alternatively, the anchor 700 can be composed of magnesium, zinc, or other bioabsorbable or dissolvable components. The anchor 700 further comprises a radially collapsed or delivery configuration (not shown) and, a radially expanded or deployed configuration **(****FIG. 11A****).** In the deployed configuration the anchor 700 is configured to radially expand and anchor itself within the IPS or CS, allowing tracking and advancement of the guard member 400, slidably disposed around the elongate guide member 780 **(****FIG. 4B****).**

The proximal portion 740 of the anchor 700 comprises a proximal marker 245, as shown in FIGS. 11A**-B.** FIGS. 12A**-B** illustrate perspective and down-the-barrel views of the proximal marker 245. The proximal marker 245 comprises an outer surface 245a including peaks and recesses, and an inner surface 245b defining an opening 245b' and a lumen 245b" **(****FIGS. 11A-12B****),** the outer 245a and inner 245b surfaces form a contour 245c of the proximal marker 245, as better appreciated in **FIG. 12B****.** The proximal marker outer surface 245a, inner surface 245b and contour 245c are configured to snuggly fit within the proximal portion 740 of the anchor 700, as shown in FIGS. 11A**-B.** The proximal marker 245 is configured to provide and maximize tensile strength when coupled to the anchor 700 and the elongate guide member 780. The proximal marker 245 is composed of tantalum or any other suitable radiopaque material. In some embodiments, the proximal marker245 is manufactured by electrode wire cutting mechanism "EDM" programmed to form the contour 245c of the proximal marker 245. The EDM manufacturing process produces the proximal marker 245 having a larger volume compared to the prior art markers, which allows smaller gaps 246 between the proximal marker 245 and the proximal portion 740 of the anchor 700, as shown in **FIG. 11B****.**

**FIG. 13** illustrates a cross-sectional view of a handle 10 for the delivery catheter 304 of **FIG. 1****,** according to embodiments of the disclosed inventions. . The handle 10 is configured to be coupled to or integrated into the delivery catheter 304, in place of the Luer assembly 17 of **FIG. 1****.** As shown in **FIG. 13****,** the handle 10 comprises a Luer fitting 12, a delivery lumen flush port 14, a tether lumen flush port 16, a tether actuation mechanism 18, a strain relief 19 and the delivery catheter 304 proximal portion 324. The delivery lumen flush port 14 is in fluid communication with the first lumen 305 of the delivery catheter 304. The tether lumen flush port 16 is an optional feature of the handle 10; the tether lumen flush port 16 is in fluid communication with the delivery catheter lumen 322 that receives the pull wire 410 for actuating the guard member 400. In some embodiments, the flush ports 14 and 16 are one way lumen flush ports. During clinical use, the flush port 14 and the flush port 16 can be connected to pressurized saline bags to maintain fluid flow through the respective delivery catheter lumens 305 and 322 to prevent blood reflux into and the formation of air bubbles within such delivery catheter lumens. The tether actuation mechanism 18 is integrated into the handle 10, for actuating guard member 400. In some embodiments, the tether actuation mechanism 18 is separate from the handle (not shown).

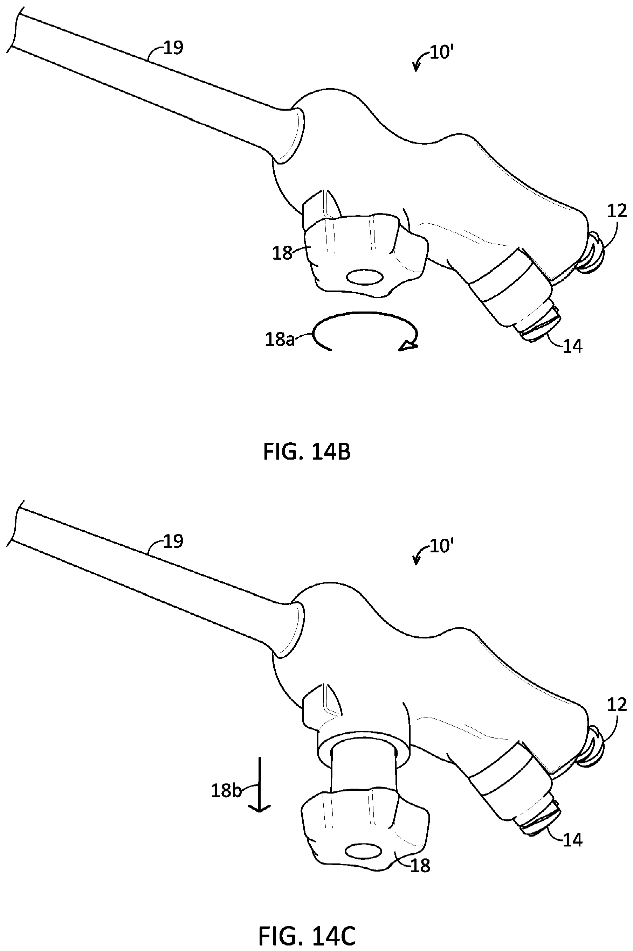

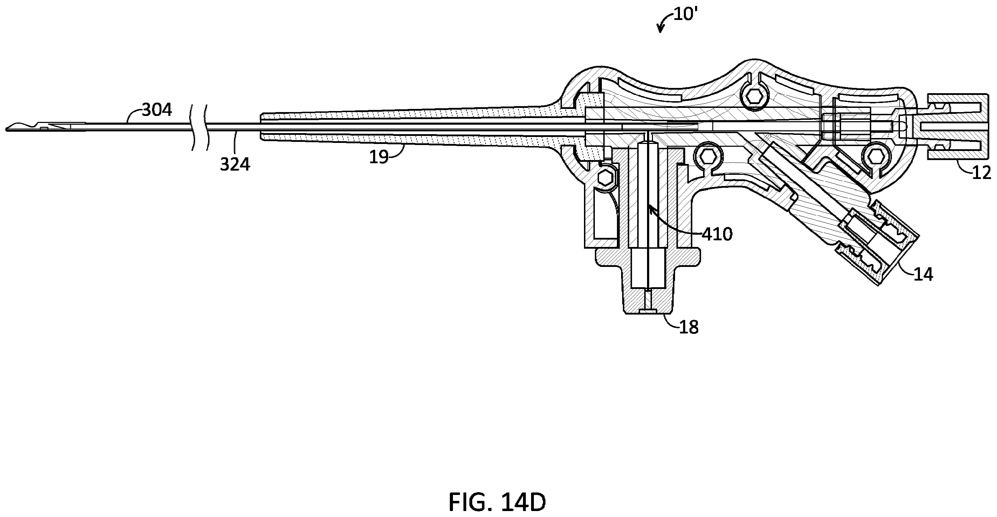

FIGS. 14A**-D** illustrate perspective and cross-sectional views of an alternative embodiment of the handle of **FIG. 13****,** according to embodiments of the disclosed inventions. As shown in FIGS. 14A**-D** the handle 10' comprises the same features as of the handle 10 (i.e., Luer fitting 12, delivery lumen flush port 14, tether retraction mechanism 18, strain relief 19 and delivery catheter 304 proximal portion 324) except that such handle 10' does not include a separate, optional, tether lumen flush port. The tether actuation mechanism 18 is configured to turn, as shown by arrow 18a in **FIG. 14B****,** so that the mechanism 18 is loosened from the handle 10' to allow translational movement 18b, as shown in **FIG. 14C****.** Further, the tether actuation mechanism 18 is configured to be pulled down, as shown by arrow 18b in **FIG. 14C****,** so as to transfer translation forces to the pull wire 410 to actuate the guard member 400 proximally and expose the penetrating element 350 from the guard member 400. It should be appreciated that the tether actuation mechanism 18 of **FIG. 13** is configured to be actuated in the way shown and described in FIGS. 14B**-D.** In addition, the tether actuation mechanism 18 of **FIG. 13** can be configured to be actuated in the opposite direction shown and described in FIGS. 14B**-D.** as to transfer translation forces to the pull wire 410 to actuate the guard member 400 distally and cover the penetrating element 350 with the guard member 400. **FIG. 14D** illustrates a cross-sectional view of the handle 10' of FIGS. 14A**-C.** As shown in **FIG. 14D****,** the tether retraction mechanism 18 is coupled to the pull wire 410. In some embodiments, the proximal end of the pull wire 410 is fixedly coupled to the tether retraction mechanism 18.

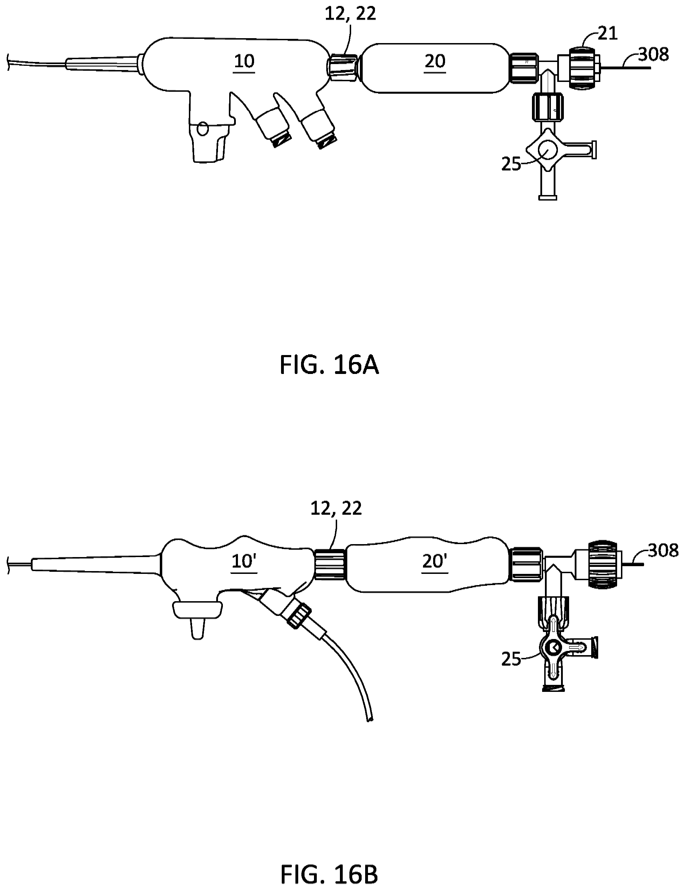

FIGS. 15A**-C** illustrate perspective and detailed views of a transfer device 20, according to embodiments of the disclosed inventions. FIGS. 15D**-G** illustrate perspective and detailed views of a transfer device 20', according to embodiments of the disclosed inventions. The transfer devices 20/20' are configured to transfer the shunt 200 and the shroud 800 into either handle 10 or 10' **(**FIGS. 16A**-B).** As shown in **FIG. 16A****,** the transfer device 20 is coupled to the handle 10, and as shown in **FIG. 1****6B****,** the transfer device 20' is coupled to the handle 10'. It should be appreciated that either transfer device 20/20' is configured to be coupled to either handle 10/10'.

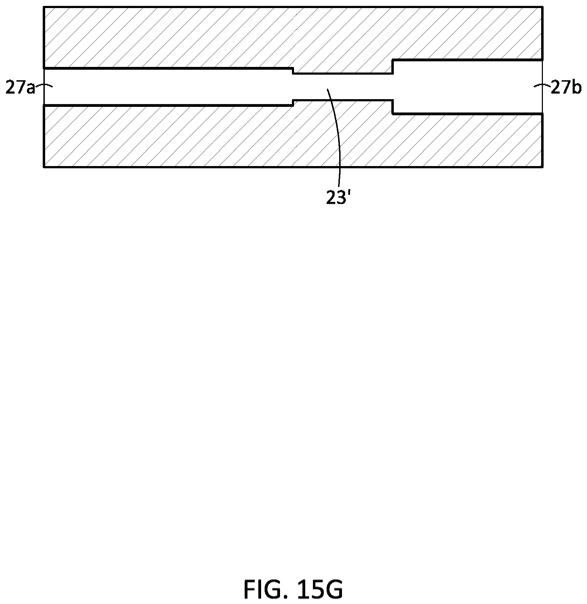

Each transfer device 20/20' comprises a touhy-borst adapter 21, a Luer fitting 22, a passive seal 23, a housing 24, and a stopcock 25, as shown in **FIGS. 15A-****B** and **15D.** The housing 24 comprises a lumen 26 extending therethrough, where the lumen 26 and passive seal lumen 23' are configured to receive the shunt 200 and the shroud 800. As shown in FIGS. 15D**-E,** the transfer device 20' comprises the same features of the transfer device 20, additionally the transfer device 20' comprises a support member 27 disposed within the housing 24. The support member 27 is configured to provide column support to the housing 24, lumen 26 and/or shroud 800 for transferring of the shunt 200 and the shroud 800 through lumen 26 and passive seal lumen 23' into either handle 10 or 10'. The support member 27 may comprise a metallic, polymeric or any other suitable material tube, elongated member, or the like. In the embodiments of FIGS. 15D**-E,** the support member 27 comprises a first support member 27a and a second support member 27b; **FIG 15G** shows a cross-sectional view of first support member 27a, passive seal 23, and second support member 27b according to the transfer device embodiment of **FIG. 15D****.** The support member 27a comprises an inner diameter (ID) between 0.0665 to 0.685 to mm; while the support member 27b comprises an ID between 1.30 to 1.42 to mm. It should be appreciated that the ID of the respective support members 27a/27b may taper between the above-identified ranges or the ID may be constant (e.g., ID of 0.675 mm for support member 27a and ID of 1.37 mm for support member 27b).

As shown in FIGS. 15D**-F,** the shunt 200 and the shroud 800 are disposed within the lumen 26 of the housing 24, where the proximal portion 202 of the shunt 200 and the proximal portion 810 of the shroud 800, including the retention feature 850, are disposed within the first support member 27a of transfer device lumen 26 (detailed view in **FIG. 15E****),** and the distal portion 204 of the shunt 200 and the anchoring mechanism 229 are disposed within the second support member 27b of transfer device lumen 26 and the passive seal lumen 23' (detailed view in **FIGS. 15F****).** In some embodiments, the support member 27a ID tapers down (e.g., tapers radially inward in a distal direction) towards the passive seal 23' of the housing 24 (shown as taper 27a' in **FIG. 15E****),** such that the retention feature 850 of the shroud 800 (shown in a relaxed/expanded configuration in **FIG. 15E****),** will assume the collapsed /contracted configuration **(****FIG. 9B** and **9E****)** as the shroud is advanced distally through the taper of support member 27a. For example, the ID of the tubular support member 27a lumen decreases in a distal direction along a length of the distal portion, and such that, as the shroud 800 and shunt 200 are advanced distally through tubular support member 27a lumen, the shroud retention feature 850 is compressed to thereby secure or grasp a proximal portion 202 of the shunt 200. The support member 27a is configured to collapse, constrain and/or restrain the protrusions 803 of retention feature 850, as the shroud 800 and shunt 200 are distally or longitudinally translated/pushed through the lumen 26 of the housing 24, thereby securing the proximal portion 202 of the shunt 200 in the retention feature 850 of the shroud 800. The passive seal lumen 23' and the ID of the support member 27b are also configured to maintained the collapsed/contracted configuration of the retention feature 850, such that the shroud 800 engages and secures the proximal portion 202 of the shunt 200 throughout the transfer through transfer device lumen 26 into the handle 10/10'. During clinical use, the touhy-borst adapter 21 is configured to open and allow distal translation of the delivery wire 308, the shroud 800 and the shunt 200 through the transfer device lumen 26 into the handle 10/10' of the delivery catheter 304. Prior to clinical use (e.g., during device shipping), the touhy-borst adapter 21 is closed around the delivery wire 308 to prevent inadvertent movement or dislodgement of the shunt 200 and the shroud 800 from transfer device 20/20'. Further, the touhy-borst adapter 21 is configured to prevent fitting 22 of the transfer device 20 is configured to be engaged and be coupled to the Luer fitting 12 of the handle 10/10', as shown in **FIGS. 16A-16B****.** The stopcock 25 is a 3-way valve configured to stop fluid flow when the stopcock 25 is disposed in a closed position. The housing 24 and the transfer device lumen 26 are configured to have the shroud 800 and shunt 200 disposed within, as shown in FIGS. 15C**-D,** and the detailed views of the housing 24 in **FIGS. 15C** and 15E**-F.** When the shroud 800 and shunt 200 are disposed within the transfer device lumen 26, the shroud 800 is disposed over the shunt 200. The anchoring mechanism 229 of the shunt 200 is in the compressed configuration within the housing 24 as shown in the detail views of **FIGS. 15C****,** **15F****;** a passive seal 23 of the housing 24 holds the anchoring mechanism 229 in the compressed configuration **(**FIGS. 15C**, 15F)** for delivery assembly 300 handling, preparation and flushing of shunt 200.

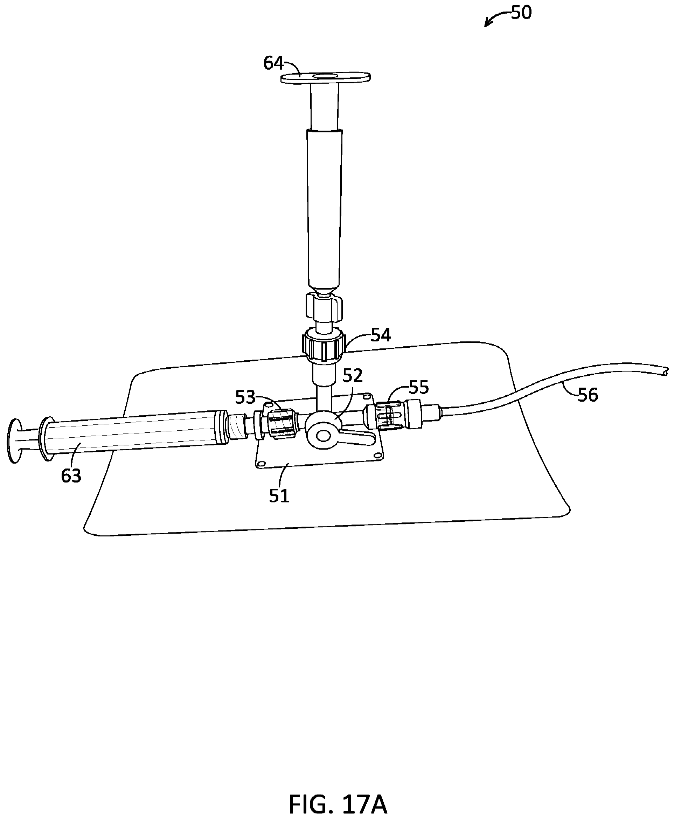

With the shunt 200 disposed within the shroud 800, the shunt 200 and shroud 800 are loaded into the transfer device 20/20'. As shown in **FIGS. 15C** and **15F****,** the distal portion 204 of the shunt 200 and the distal portion 830 of the shroud 800 are disposed partially within the passive seal lumen 23', within the fluid path of the transfer device lumen 26. The passive seal 23 is configured to seal around a portion of the distal anchoring mechanism 229 and the distal portion 204 of the shunt 200 **(****FIG. 15F****)** to facilitate fluid flushing through the shunt lumen 207 and fluid flow from reservoir 50 (described below in FIGS. 17A**-B)** while confirming patency of the shunt valve 209 while preparing the shunt 200 for implantation. The passive seal lumen 23' provides an interference fit with the distal anchoring mechanism 229 and the distal portion 204 of shunt 200 such that fluid entering the transfer device lumen 26 via Luer fitting 22 is directed into the shunt lumen 207 and minimizes fluid flow around the exterior surfaces of the distal anchoring mechanism 229 and the shunt body 203.

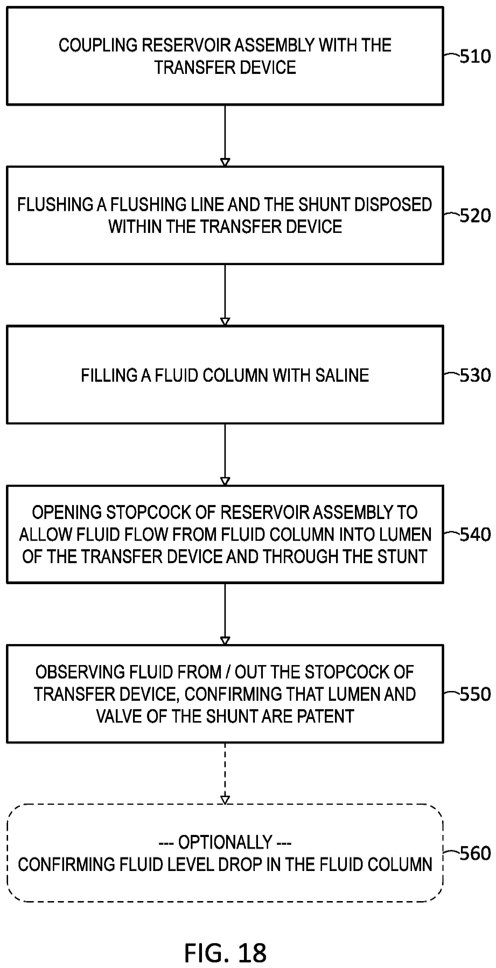

The clinician may complete a patency test 500 (FIG. 18) when the shroud 800 and shunt 200 are disposed within the transfer device 20/20', then, confirm flow out of the stopcock 25 (e.g., prior to implantation of the shunt 200), as further described in FIGS. 17A**-B)**

Following valve patency test 500, the clinician couples the transfer device 20 to the handle 10/10' **(**FIGS. 16A**-B)** and distally translates (i.e., pushes) the shroud 800 and the shunt 200 using the delivery wire 308 from the passive seal 23 into and through lumen 26 **(**FIGS. 15B**-G** and 16A-B) of the transfer device 20/20' into the handle 10/10', and further into the lumen 305 of the delivery catheter 304. As previously disclosed, the shroud 800 translates the shunt 200 through the handle 10/10' and into the delivery catheter lumen 305 through the shroud distal radiopaque marker 862 pushing against the shunt engagement region 244 and the proximal retention feature 850 pushing against the shunt proximal portion 202. The transfer device lumen 26 is in fluid communication with the handle 10/10'. The clinician may use the markers of the shroud 800 and/or the delivery wire 308 to assist with the positioning of the shroud 800 and the shunt 200 within the handle 10/10', so that the delivery assembly 300 is suitable for the medical procedure. The clinician can continue advancing the shroud 800 and the shunt 200 into a distal portion of delivery catheter first lumen 305 via delivery wire 308. One or more markings on a proximal portion of the delivery wire 308 are configured to indicate when the delivery wire 308 has been sufficiently advanced, such that the shunt 200 and the shroud 800 are advanced into a distal portion of lumen 305, which provides additional column strength and resistance to kinking in the distal portion 344 of delivery catheter 304 during the implantation procedure.

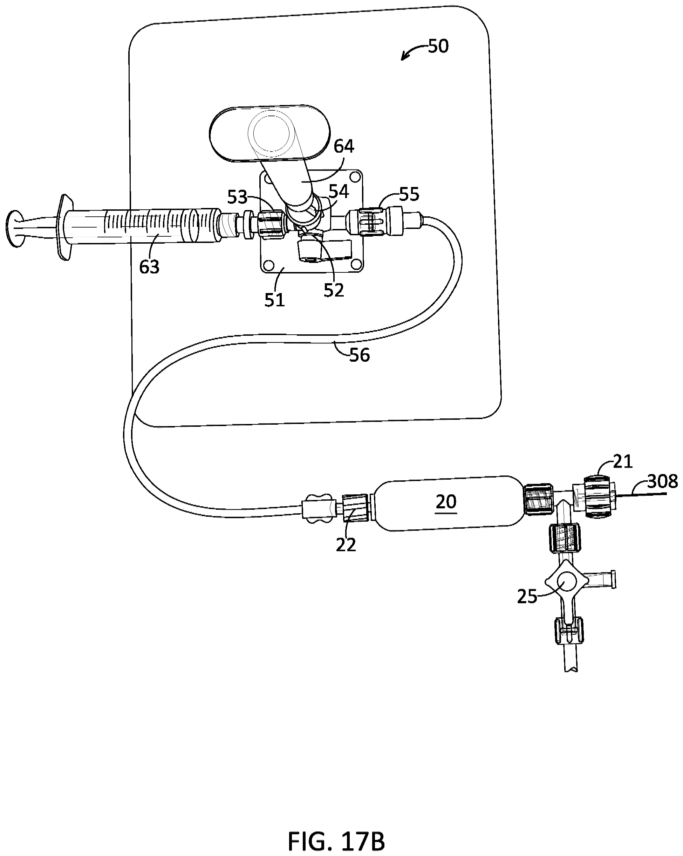

**FIG. 17A** illustrates a perspective view of a reservoir assembly 50, according to embodiments of the disclosed inventions. **FIG. 17B** illustrates a top view of the reservoir assembly 50 and a perspective view of the transfer device 20/20', according to embodiments of the disclosed inventions. As shown in **FIGS. 17A**-B, the reservoir assembly 50 comprises a 3-way stopcock 52 having a plurality of Luer fittings 53, 54 and 55; the stopcock 52 is secured into a base 51. Base 51 includes a hinged or swivel mechanism coupled to stopcock 52; reservoir assembly 50 is packaged with fluid column 64 lying parallel to the die card surface of **FIG. 17****;** when a clinician or technician is ready to prepare shunt 200 for clinical use, the reservoir assembly 90 is rotated 90 degrees upward from the die card until the base 51 provides a positive lock to hold reservoir assembly 50 and fluid column 64 upright as shown in **FIG. 17A****;** the positive lock is achieved by an interference fit of the molded hinge components in base 51 (not shown).

As shown in **FIG. 17B****,** the reservoir assembly 50 is in fluid communication with the Luer 22 and lumen 26 of the transfer device 20, via a flushing line 56 of the reservoir assembly 50. The Luer fitting 53 of the reservoir assembly 50 is coupled to a syringe 63 to allow fluid flushing through the stopcock 52 and Luer fitting 55, into the flushing line 56 connected to the transfer device 20 into the transfer device lumen 26. The stopcock 52 can open to allow fluid flow from the syringe 63 for flushing of the flushing line 56 connected to the transfer device 20. The fluid flushing from the reservoir assembly 50 into the transfer device 20/20' is configured to flush fluid through shunt lumen 207 via the passive seal 23 to remove air bubbles that can prevent, decrease fluid flow or increase backflow through valve 209 of the shunt 200 (not shown). Additionally, the Luer fitting 54 of the reservoir assembly 50 is coupled to a fluid column 64. The stopcock 52 can open to fill the column 64 with fluid (e.g., saline, heparinized saline, sterile water); and the stopcock 52 can open to allow fluid to flow from the fluid column 64 into transfer device 20 via the flushing line 56. With the fluid column 64 filled and the stopcock 52 open to the transfer device 20/20', fluid is allowed to flow from the column 64 through the flushing line 56 into transfer device 20/20' and the transfer device lumen 26. The passive seal 23 of the housing 24 of the transfer device 20/20' holds the shunt anchoring mechanism 229 in the compressed configuration and provides a fluid seal around the shunt 200 disposed in the lumen 26 of the transfer device 20/20' **(****FIG. 15C** and 15F**-G).** The passive seal 23 directs fluid flow received from the flushing line 56 through the lumen 26 into the distal portion of shunt lumen 207, out of shunt valve 209, the transfer device lumen 26 and out of the stopcock 25 of the transfer device 20. Fluid column 64 can be filled to a height known to provide a desired or known amount of fluid pressure on valve 209 (via flushing line 56, transfer device lumen 26 and passive seal lumen 23').

**FIG. 18** illustrates a patency test 500 for confirming patency of the shunt 200, according to the embodiments of the inventions. The patency test 500 provides confirmation that the shunt 200 and the shunt valve 209 will provide a therapeutic flow rate of CSF (e.g., more than 5 mL/hr.) from the SAS to the venous system at a target differential pressure range between the SAS and venous system after the shunt deployment procedure. The transfer device 20/20' **(**FIGS. 15A**-G** and **16A-B)** and reservoir assembly 50 **(**FIGS. 17A**-B)** are configured to be used in the manner, actions, and/or steps described in the patency test 500 of **FIG. 18****.** The patency test 500 comprises: coupling 510 the reservoir assembly 50 with the transfer device 20/20' through the flushing line 56; flushing 520 (e.g., at least one time or preferably two or more times), the flushing line 56 and the shunt 200 disposed within the transfer device 20 with saline or heparinized saline via the syringe 63; filling 530 the fluid column 64 with saline to a height that correlates with an expected differential pressure between the SAS and venous system; opening 540 the stockcock 52 to allow fluid flow from the fluid column 64 to the flushing line 56 into the lumen 26 of the transfer device 20 and through the shunt 200 (e.g., into CSF lumen 207 and out valve 209); observing 550 fluid flow from/out of 3-way stopcock 25 of transfer device 20/20', confirming that lumen 207 and valve 209 of the shunt 200 are patent. Optionally, confirming 560 that the fluid level in the fluid column 64 drops a predetermined distance (e.g., 1 cm) with a specified time period (e.g., five minutes) during which fluid flows from the fluid column 64 into the flushing line 56 and transfer device 20/20' and out of shunt 200. The predetermined fluid drop in fluid column 64 from a specified fluid height in fluid column 64 over a specified time period can be correlated to known fluid flow rates for a range of fluid pressures applied through shunt lumen 207 and valve 209 to provide an indicator of therapeutic performance and anticipated CSF flow rates through shunt 200 during clinical use of the device.

Using the transfer device 20/20' and reservoir assembly 50, for example, as described in the patency test 500, eliminates the prior art need for the clinician to advance or proximally withdraw the shunt 200 into delivery catheter 304 through the penetrating element 350; avoiding the risk of the penetrating element 350 skiving, tearing, or damaging the shunt 200 including the shunt lumen 207 and the shunt valve 209 during loading. Further, the use of the transfer device 20/20' and reservoir assembly 50 eliminates the prior art need for a clinician to directly handle the shunt 200 during device preparation for clinical use and the implantation procedure; the direct handing of the shunt 200 or conventional shunt devices implanted through open surgical procedures increases the risk of compromising the device sterility and introducing contaminants onto the shunt 200 before the deployment procedure which can cause patient infection. Additionally, the transfer device 20/20' eliminates the need to directly handle the shunt 200 through all of the preparation for clinical use steps such as flushing, patency test 500, and delivery catheter loading.

Further, it should be appreciated that the transfer device 20/20' lumen 26 and the first lumen 305 of the delivery catheter 304 should have substantially similar diameters, such that there is no need for the shroud 800 to accommodate stretching the shunt 200 and necking down the outer diameter of shunt 200 while loading the shunt 200 into delivery catheter 304. Stretching the shunt 200 creates the potential for damaging the polymer shunt body and/or biocompatible coating on the exterior surface of the shunt 200 and/or valve 209. Stretching of the shunt 200 was needed in prior art systems, which requires a complex shroud design to maintain control over the shunt 200 within delivery catheter first lumen 305 and significantly increases the friction between shunt 200 and first lumen 305 during the implantation procedure. In addition to the complex shroud design, the prior art systems relied on a complex apparatus for flushing shunt 200, stretching shunt 200, and loading shunt 200 through delivery catheter penetrating element 350 into delivery catheter first lumen 305, for example, as disclosed in U.S. Patent Application Publication No. US20210228846. Further, the prior art need for stretching of the shunt 200 required the clinician to overcome the resistance between the shunt 200 and first lumen 305 of the delivery catheter 304, while advancing the shunt 200 through first lumen 305 and out of the penetrating element 305 during the shunt deployment procedure. The act of overcoming said resistance can reduce important tactile feedback received by the clinician during the shunt deployment procedure. Embodiments of the delivery catheter 304, shroud 800 and transfer device 20 disclosed herein overcome the drawbacks stemming from shunt stretching, decrease resistance to shunt advancement, and provide an overall smoother and more controlled deployment of shunt 200.

Embodiments of delivery catheter 304 have been disclosed for navigation and deployment of shunt 200. Delivery catheter 304 can also be used in other procedures where it is necessary to access and/or deploy devices into the SAS of a patient. For example, embodiments of delivery catheter 304 can be used to access the SAS and deliver a therapeutic agent from the delivery catheter first lumen 305. Alternatively, a micro catheter can be navigated through the delivery catheter first lumen 305 and penetrating element 350 to a location or region within the SAS, and a therapeutic agent can be delivered through the micro catheter into the SAS. Nonlimiting examples of therapeutic agents that can be delivered to the SAS with embodiments of delivery catheter 304 can include compositions comprising anti-sense RNA or anti-sense oligonucleotides, anti-bodies, anti-biotics, anti-vasospasm agents, biosimilars, chemotherapy agents, GABA receptor agonists, therapies intended for the treatment of neurodegenerative diseases (e.g., Alzheimer's disease, Parkinson's disease, Huntington's disease, prion disease, motor neurone diseases, spinocerebellar ataxia, and spinal muscular atrophy), therapies intended for the treatment of trigeminal neuralgia, therapies intended for the treatment of pontine glioma, tissue plasminogen activator, and any other composition(s) intended to have a therapeutic effect on all or a portion(s) of the central nervous system.

Although particular embodiments have been shown and described herein, it will be understood by those skilled in the art that they are not intended to limit the present inventions, and it will be obvious to those skilled in the art that various changes, permutations, and modifications may be made (e.g., the dimensions of various parts, combinations of parts) without departing from the scope of the disclosed inventions, which is to be defined only by the following claims and their equivalents. The specification and drawings are, accordingly, to be regarded in an illustrative rather than restrictive sense. The various embodiments shown and described herein are intended to cover alternatives, modifications, and equivalents of the disclosed inventions, which may be included within the scope of the appended claims.
The following numbered clauses set out certain aspects of the present application:
1. A delivery system for deployment of an implant, the delivery system comprising:
   a delivery catheter, the delivery catheter comprising a first delivery catheter lumen extending from an open distal end of the delivery catheter into a handle coupled to a proximal end portion of the delivery catheter;
   a tissue penetrating element disposed on, and extending distally from, an open distal end of the delivery catheter; and
   a guard member comprising a proximal portion disposed over, and translatable relative to, the respective tissue penetrating element and distal end of the delivery catheter, the guard member defining a guard member lumen configured to receive and cover the tissue penetrating element, the guard member lumen defining a longitudinal axis of the guard member,
   wherein the proximal portion of the guard member transitions into a split-open distal portion adjacent a distal opening of the guard member lumen, the split-open distal portion comprising a contour configured to engage and deflect the tissue penetrating element at an angle relative to the longitudinal axis of the guard member when the guard member is translated proximally relative to the tissue penetrating element, and
   wherein the split-open distal portion of the guard member comprises arcuate sloping portions on opposing sides of the contour, the sloping portions having respective surfaces that are configured to engage and deflect the tissue penetrating element.
2. The delivery system of clause 1, wherein the contour of the split-open distal portion of the guard member is configured to allow the respective sloping portions of the opposing sides to open and collapse around the tissue penetrating element when the respective guard member and delivery catheter are retracted into a guide catheter.
3. The delivery system of clause 1 or 2, further comprising a pull wire coupled to the guard member and configured to translate the guard member proximally relative to the tissue penetrating element.
4. The delivery system of clause 3, wherein a distal portion of the pull wire bifurcates into respective first and second pull wire members.
5. The delivery system of clause 4, further comprising an arcuate radiopaque marker embedded within the guard member adjacent the distal opening of the guard member lumen, wherein the first and second pull wire members are each attached to the arcuate radiopaque marker.
6. The delivery system of clause 5, the guard member lumen comprising a first guard member lumen, the guard member further comprising a second guard member lumen that spans between the proximal and distal portions of the guard member, wherein the second guard member lumen is configured to receive an elongate guide member therethrough over which the guard member translates, and wherein the second guard member lumen is partially surrounded by the arcuate marker.
7. The delivery system of clause 3,
   wherein the delivery catheter further comprises a second delivery catheter lumen, wherein the pull wire is disposed in the second delivery catheter lumen, and
   wherein the handle further comprises
      a flush port in fluid communication with the first catheter lumen, and
      a tether actuation mechanism, wherein the proximal end of the pull wire is coupled to the tether actuation mechanism, and wherein the tether actuation mechanism is configured to be pulled relative to the handle to thereby translate the guard member relative to the tissue penetrating element to thereby expose the tissue penetrating element.
8. The delivery system of clause 3, wherein the distal portion of the pull wire is embedded within the guard member.
9. The delivery system of any of clauses 1-3 and 7-8, further comprising an arcuate radiopaque marker embedded within the guard member adjacent the distal opening of the guard member lumen.
10. The delivery system of a clause 9, the guard member lumen comprising a first guard member lumen, the guard member further comprising a second guard member lumen that spans between the proximal and distal portions of the guard member, wherein the second guard member lumen is configured to receive an elongate guide member therethrough over which the guard member translates, and wherein the second guard member lumen is partially surrounded by the arcuate marker.
11. The delivery system of clause 9, further comprising an elongate radiopaque marker spine embedded in the tissue penetrating element,
   wherein the marker spine is aligned substantially parallel to a longitudinal axis of the tissue penetrating element, and
   wherein, when the tissue penetrating element is disposed within the guard member lumen, the arcuate radiopaque marker embedded in the guard member and the radiopaque marker spine embedded in the tissue penetrating element are configured to indicate a position, orientation and trajectory of the tissue penetrating element.
12. A delivery system for deployment of an implant, the delivery system comprising:
   a delivery catheter having a proximal opening, a distal opening, and delivery catheter lumen extending therebetween; and
   a shroud assembly, the shroud assembly comprising
      an elongate tubular shroud body having open proximal and distal ends;
      an implant retention feature coupled to the proximal end of the shroud body, the implant retention feature having a proximal end and an open distal end; and
      a shroud lumen extending through the shroud body from the distal end opening of the shroud body, the proximal end opening of the shroud body, and an interior of the implant retention feature, respectively,
      wherein the delivery catheter lumen is configured to receive the shroud assembly therein and allow passage of the shroud assembly therethrough, and
      wherein the implant retention feature is configured to secure a proximal end portion of an implant in the shroud assembly when the implant is disposed in the shroud lumen and the shroud assembly is disposed in the delivery catheter lumen.
13. The delivery system of clause 12, wherein the implant retention feature is configured to release the proximal end portion of the implant when the shroud assembly is advanced out of the delivery catheter lumen through the distal opening of the delivery catheter.
14. The delivery system of clause 12 or 13, further comprising a delivery wire coupled to the proximal end of the implant retention feature and configured to advance or retract the shroud assembly within the delivery catheter lumen with the implant disposed within the shroud lumen.
15. The delivery system of any of clauses 12-14, wherein the implant retention feature comprises a plurality of elongated members extending between two annular members, wherein the elongated members comprise respective protrusions facing radially outward, such that, when the implant is disposed within the shroud lumen, and the shroud assembly is disposed within the delivery catheter lumen, the protrusions are compressed by an interior wall of the delivery catheter lumen and the elongated members are in-turn compressed against the proximal end portion of the implant to thereby grasp the implant within the shroud lumen.
16. The delivery system catheter of any of clauses 12-15, wherein the shroud body comprises a spiral cut hypotube and an uncut axial spine disposed along a length of the hypotube.
17. The delivery system of any of clauses 12-16, wherein the shroud assembly further comprises a proximal radiopaque marker disposed at the proximal end of the implant retention feature, and a distal radiopaque marker disposed at the distal end opening of the shroud body.
18. The delivery system of clause 17, wherein an annular edge of the distal radiopaque maker is configured to contact an annular edge of an implant distal anchoring mechanism in order to advance the implant through the delivery catheter lumen and out the distal end opening of the delivery catheter.
19. A transfer device for flushing and transferring an implant into a delivery catheter, the implant transfer device comprising:
   an elongate housing;
   an implant transfer lumen extending through the housing from a proximal opening in the housing to a distal opening in the housing;
   a distal luer connector coupled to the distal opening; a touhy-borst adapter and stopcock coupled to the proximal opening; and
   an annular passive seal disposed within the implant transfer lumen, wherein the annular passive seal comprises a lumen configured to retain an expandable portion of the implant in a compressed configuration, and wherein the distal luer connector, implant transfer lumen, passive seal lumen, touhy-borst adapter and stopcock are in fluid communication.
20. The transfer device of clause 19, further comprising a shroud assembly disposed in the implant transfer lumen, the shroud assembly comprising:
   an elongate tubular shroud body having open proximal and distal ends;
   an implant retention feature coupled to the proximal end of the shroud body, the implant retention feature having a proximal end and an open distal end; and
   a shroud lumen extending through the shroud body from the distal end opening of the shroud body, the proximal end opening of the shroud body, and an interior of the implant retention feature, respectively,
   wherein a body portion of the implant is disposed within the shroud lumen, a proximal portion of the implant is disposed within the retention feature, and a distal anchoring mechanism of the implant is disposed in a compressed configuration within the passive seal lumen, and
   wherein the transfer device distal luer connector, implant transfer lumen, passive seal lumen, a lumen of the implant, and transfer device touhy-borst adapter and stopcock are in fluid communication.
21. The transfer device of clause 20, the shroud assembly further comprising a delivery wire coupled to the proximal end of the implant retention feature and configured to advance or retract the shroud assembly within the implant transfer lumen with the implant disposed within the shroud lumen.
22. The transfer device of clause 20 or 21, wherein the implant retention feature comprises a plurality of elongated members extending between two annular members, wherein the elongated members comprise respective protrusions facing radially outward.
23. The transfer device of any of clauses 20-22, wherein the shroud body comprises a spiral cut hypotube and an uncut axial spine disposed along a length of the hypotube.
24. The transfer device of any of clauses 20-23, wherein the shroud assembly further comprises a proximal radiopaque marker disposed at the proximal end of the implant retention feature, and a distal radiopaque marker disposed at the distal end opening of the shroud body.
25. The transfer device of any of clauses 20-24, wherein the transfer device further comprises a tubular support member disposed in the implant transfer lumen, the tubular support member defining an inner lumen through which the shroud assembly and implant carried in the shroud assembly are advanced, wherein a distal portion of the tubular support member tapers radially inward in a distal direction, such that an inner diameter of the tubular support member lumen decreases in a distal direction along a length of the distal portion, and such that, as the shroud assembly And implant are advanced distally through tubular support member lumen, the shroud retention feature is compressed to thereby secure or grasp a proximal end portion of the implant.
26. An assembly for verifying patency of a shunt implant prior to clinical use, the assembly comprising:
   a base;
   a three-way stopcock secured to the base, the stopcock having a plurality of luer fittings;
   a first stopcock luer fitting connectable to a fluid source;
   a fluid column in fluid communication with a second stopcock luer fitting; and
   a flushing line comprising a distal end opening in fluid communication with a third stopcock luer fitting, the flushing line further comprising a proximal end opening.
27. The assembly of clause 26, wherein the stopcock has a first open position that places the fluid source in fluid communication with the flushing line, a second open position that places the fluid source in fluid communication with the fluid column, and a third open position that places the fluid column in fluid communication with the flushing line.
28. The assembly of clause 26 or 27, wherein the fluid source comprises a syringe.
29. The assembly of any of clauses 26-28, wherein the base comprises a hinge mechanism secured to the stopcock.
30. The assembly of clause 29, wherein the hinge mechanism is configured to swivel the stopcock and fluid column from a first position in which the fluid column is disposed substantially parallel to a surface of the base, to a second position in which the fluid column is disposed substantially perpendicular to the base surface.
31. The assembly of clause 30, wherein the hinge mechanism is configured to releasably secure the stopcock in the second position.
32. A method for preparing a shunt implant for clinical use, the method comprising:
   connecting the flushing line of the assembly of clause 26 to the distal luer connector of the implant transfer device of clause 22, such that the flushing line is in fluid communication with a lumen of the shunt;
   placing the assembly stopcock in the first open position; and
   delivering fluid from the fluid source through the respective flushing line and shunt lumen.
33. The method of clause 32, further comprising:
   placing the assembly stopcock in the second open position; and
   delivering fluid from the fluid source into the fluid column.
34. The method of clause 33, further comprising:
   placing the assembly stopcock in the third open position; and
   confirming fluid flow from the fluid column through the respective flushing line and shunt lumen by observing fluid flowing out of the implant transfer device stopcock.
35. The method of clause 32 or 34, wherein the passive annular seal directs fluid flow in the implant transfer device lumen into the shunt lumen.
36. The method of clause 34 or 35, further comprising:
   disconnecting the flushing line from the distal luer connector of the implant transfer device;
   connecting the distal luer connector of the implant transfer device to the proximal luer connector of a delivery catheter handle; and
   advancing the shunt implant through the implant transfer device lumen and into a lumen of the delivery catheter.
37. The method of any of clauses 32-36, wherein the fluid source comprises a syringe.
38. The method of any of clauses 32-36, wherein the fluid comprises heparinized saline.
39. The method of clause 36, wherein advancing the shunt implant through the implant transfer device lumen and into a lumen of a delivery catheter comprises advancing the implant and shroud assembly with the pusher wire.
40. The method of clause 39, wherein when advancing the implant and shroud assembly from the transfer device lumen into the delivery catheter lumen, a plurality of protrusions of the proximal retention feature compress the retention feature elongate members to grasp the proximal portion of the implant.
41. The method of clause 39 or 40, wherein when advancing the implant and shroud assembly through the transfer device lumen and into the delivery catheter lumen, an annular edge of the shroud distal radiopaque marker of clause 25 contacts an annular edge of an implant distal anchoring mechanism to advance the implant through the transfer tool lumen into the delivery catheter lumen.
42. A catheter comprising:
   a first catheter lumen extending from an open distal end of the catheter into a handle coupled to a proximal end portion of the catheter;
   a tissue penetrating element disposed on, and extending distally from, an open distal end of the catheter; and
   a guard member comprising a proximal portion disposed over, and translatable relative to, the respective tissue penetrating element and distal end of the catheter, the guard member defining a guard member lumen configured to receive and cover the tissue penetrating element, the guard member lumen defining a longitudinal axis of the guard member,
   wherein the proximal portion of the guard member transitions into a split-open distal portion adjacent a distal opening of the guard member lumen, the split-open distal portion comprising a contour configured to engage and deflect the tissue penetrating element at an angle relative to the longitudinal axis of the guard member when the guard member is translated proximally relative to the tissue penetrating element.
43. The catheter of clause 42, wherein the split-open distal portion of the guard member comprises arcuate sloping portions on opposing sides of the contour, the sloping portions having respective surfaces that are configured to engage and deflect the tissue penetrating element.
44. The catheter of clause 43, wherein the contour of the split-open distal portion of the guard member is configured to allow the respective sloping portions of the opposing sides to open and collapse around the tissue penetrating element when the respective guard member and catheter are retracted into a guide catheter.
45. The catheter of any of clauses 42-44, further comprising a pull wire coupled to the guard member and configured to translate the guard member proximally relative to the tissue penetrating element.
46. The catheter of clause 45, wherein a distal portion of the pull wire bifurcates into respective first and second pull wire members.
47. The catheter of clause 46, further comprising an arcuate radiopaque marker embedded within the guard member adjacent the distal opening of the guard member lumen, wherein the first and second pull wire members are each attached to the arcuate radiopaque marker.
48. The catheter of clause 47, the guard member lumen comprising a first guard member lumen, the guard member further comprising a second guard member lumen that spans between the proximal and distal portions of the guard member, wherein the second guard member lumen is configured to receive an elongate guide member therethrough over which the guard member translates, and wherein the second guard member lumen is partially surrounded by the arcuate marker.
49. The catheter of clause 45,
   wherein the catheter further comprises a second catheter lumen, wherein the pull wire is disposed in the second delivery catheter lumen, and
   wherein the handle further comprises
      a flush port in fluid communication with the first catheter lumen, and
      a tether actuation mechanism, wherein the proximal end of the pull wire is coupled to the tether actuation mechanism, and wherein the tether actuation mechanism is configured to be pulled relative to the handle to thereby translate the guard member relative to the tissue penetrating element to thereby expose the tissue penetrating element.
50. The delivery system of clause 46, wherein the distal portion of the pull wire is embedded within the guard member.
51. The catheter of any of clauses 42-46, further comprising an arcuate radiopaque marker embedded within the guard member adjacent the distal opening of the guard member lumen.
52. The catheter of clause 51, the guard member lumen comprising a first guard member lumen, the guard member further comprising a second guard member lumen that spans between the proximal and distal portions of the guard member, wherein the second guard member lumen is configured to receive an elongate guide member therethrough over which the guard member translates, and wherein the second guard member lumen is partially surrounded by the arcuate marker.
53. The catheter of clause 52, further comprising an elongate radiopaque marker spine embedded in the tissue penetrating element, wherein the marker spine is aligned substantially parallel to a longitudinal axis of the tissue penetrating element, and wherein, when the tissue penetrating element is disposed within the guard member lumen, the arcuate radiopaque marker embedded in the guard member and the radiopaque marker spine embedded in the tissue penetrating element are configured to indicate a position, orientation and trajectory of the tissue penetrating element.
54. A shroud assembly for delivering an implant through a catheter lumen, the shroud assembly comprising:
   an elongate tubular shroud body having open proximal and distal ends;
   an implant retention feature coupled to the proximal end of the shroud body, the implant retention feature having a proximal end and an open distal end; and
   a shroud lumen extending through the shroud body from the distal end opening of the shroud body, the proximal end opening of the shroud body, and an interior of the implant retention feature, respectively, and
   wherein the implant retention feature is configured to secure a proximal end portion of the implant in the shroud assembly when the implant is disposed in the shroud lumen and the shroud assembly is disposed in the catheter lumen.
55. The shroud assembly of clause 54, wherein the implant retention feature is configured to release the proximal end portion of the implant when the shroud assembly is advanced out of a distal end opening of the catheter lumen.
56. The shroud assembly of clause 54 or 55, further comprising a delivery wire coupled to the proximal end of the implant retention feature and configured to advance or retract the shroud assembly within the delivery catheter lumen with the implant disposed within the shroud lumen.
57. The shroud assembly of any of clauses 54-56, wherein the implant retention feature comprises a plurality of elongated members extending between two annular members, wherein the elongated members comprise respective protrusions facing radially outward, such that, when the implant is disposed within the shroud lumen, and the shroud assembly is disposed within the catheter lumen, the protrusions are compressed by an interior wall of the catheter lumen and the elongated members are in-turn compressed against the proximal end portion of the implant to thereby grasp the implant within the shroud lumen.
58. The shroud assembly of any of clauses 54-57, wherein the shroud body comprises a spiral cut hypotube and an uncut axial spine disposed along a length of the hypotube.
59. The shroud assembly of any of clauses 54-58, wherein the shroud assembly further comprises a proximal radiopaque marker disposed at the proximal end of the implant retention feature, and a distal radiopaque marker disposed at the distal end opening of the shroud body.
60. The delivery system of any of clauses 3-5 or 7-8, wherein the pull wire comprises a rectangular, circular, or crescent cross-sectional profile.
61. The catheter of any of clauses 45-47 or 49-50, wherein the pull wire comprises a rectangular, circular, or crescent cross-sectional profile.

## Claims

1. A shroud assembly for delivering an implant through a catheter lumen, the shroud assembly comprising:
an elongate tubular shroud body having open proximal and distal ends;
an implant retention feature coupled to the proximal end of the shroud body, the implant retention feature having a proximal end and an open distal end; and
a shroud lumen extending through the shroud body from the distal end opening of the shroud body, the proximal end opening of the shroud body, and an interior of the implant retention feature, respectively, and
wherein the implant retention feature is configured to secure a proximal end portion of the implant in the shroud assembly when the implant is disposed in the shroud lumen and the shroud assembly is disposed in the catheter lumen.

2. The shroud assembly of claim 1, wherein the implant retention feature is configured to release the proximal end portion of the implant when the shroud assembly is advanced out of a distal end opening of the catheter lumen.

3. The shroud assembly of claim 1 or 2, further comprising a delivery wire coupled to the proximal end of the implant retention feature and configured to advance or retract the shroud assembly within the delivery catheter lumen with the implant disposed within the shroud lumen.

4. The shroud assembly of any of claims 1-3, wherein the implant retention feature comprises a plurality of elongated members extending between two annular members, wherein the elongated members comprise respective protrusions facing radially outward, such that, when the implant is disposed within the shroud lumen, and the shroud assembly is disposed within the catheter lumen, the protrusions are compressed by an interior wall of the catheter lumen and the elongated members are in-turn compressed against the proximal end portion of the implant to thereby grasp the implant within the shroud lumen.

5. The shroud assembly of any of claims 1-4, wherein the shroud body comprises a spiral cut hypotube and an uncut axial spine disposed along a length of the hypotube.

6. The shroud assembly of any of claims 1-5, wherein the shroud assembly further comprises a proximal radiopaque marker disposed at the proximal end of the implant retention feature, and a distal radiopaque marker disposed at the distal end opening of the shroud body.
